(19) Europäisches Patentamt
European Patent Office
Office européen des brevets

(11) Numéro de publication: **0 434 561 B1**

(12) # FASCICULE DE BREVET EUROPEEN

(45) Date de publication du fascicule du brevet: **18.10.95**

(21) Numéro de dépôt: **90403688.6**

(22) Date de dépôt: **20.12.90**

(51) Int. Cl.[6]: **C07D 295/12**, A61K 31/495, C07D 213/82, A61K 31/44, C07C 209/48, A61K 31/40, C07D 333/38, A61K 31/38, C07D 487/04, //(C07D487/04, 239:00,235:00)

(54) **Dérivés de la napht-1-yl pipérazine, leur procédé de préparation et les compositions pharmaceutiques qui les contiennent.**

(30) Priorité: **20.12.89 FR 8916882**

(43) Date de publication de la demande:
**26.06.91 Bulletin 91/26**

(45) Mention de la délivrance du brevet:
**18.10.95 Bulletin 95/42**

(84) Etats contractants désignés:
**AT BE CH DE DK ES FR GB GR IT LI LU NL SE**

(56) Documents cités:
**EP-A- 0 000 220**
**EP-A- 0 104 614**
**EP-A- 0 343 050**
**EP-A- 0 372 657**
**GB-A- 721 417**

(73) Titulaire: **ADIR ET COMPAGNIE**
**1 rue Carle Hébert**
**F-92415 Courbevoie Cédex (FR)**

(72) Inventeur: **Lavielle, Gilbert**
**1 avenue Lilly**
**F-78170 La Celle Saint Cloud (FR)**
Inventeur: **Laubie, Michel**
**35 Avenue Foch**
**F-92420 Vaucresson (FR)**
Inventeur: **Colpaert, Francis**
**36 bis boulevard Carnot**
**F-78110 Le Vesinet (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

JOURNAL OF MEDICINAL CHEMISTRY, vol. 31, no. 10, 1988, pages 1969-1971, Washington, US; R.A. GLENNON et al.: "Arylpiperazine derivatives as high-affinity 5-HT1A serotonin ligands"

Drug Development Research 2 (21-48) 1992.

European Journal of Pharmacology and Experimental Therapeutics, 203 (1991) p. 319-322

Journal of Pharmacology and Experimental Therapeutics, vol. 262, no. 2 p. 451-463 (1992)

## Description

La présente invention a pour objet des nouveaux dérivés de la napht-1-yl pipérazine, leur procédé de préparation et les compositions pharmaceutiques qui les contiennent.

Certains dérivés de la napht-1-yl-4-alkyl pipérazine, ayant des propriétés neuroleptiques ou antipsychotiques, sont décrits dans le brevet US 4831031 et dans les demandes de brevet EP 281309 et EP 279598. Des dérivés de la 1-[3-(3,4,5-triméthoxyphénoxy)-2-hydroxypropyl]-4-aryl-pipérazine ayant une activité antiagressive sont décrits dans le brevet US 4335126. Des dérivés des arylpipérazines ayant une activité antagoniste au niveau des récepteurs 5-$HT_{1A}$ sont décrits dans J.Med.Chem.,(1989),32, p.1921-1926 et Drug Dev.Res.,(1989),16,p.335-343. Il est aussi connu que la napht-1-yl pipérazine est un ligand des récepteurs à la sérotonine (Journal of Receptor Research,(1988),8,(1-4),p.59-81).

Les composés de l'invention se distinguent des autres dérivés de la napht-1-yl pipérazine décrits dans la littérature par leurs structures originales et par leurs propriétés pharmacologiques.

Au niveau cardiovasculaire, les composés de l'invention diminuent la pression artérielle et la fréquence cardiaque. Cette action résulte d'une inhibition centrale du tonus sympathique et elle est reliée à leurs propriétés agonistes 5-$HT_{1A}$. Au niveau du système nerveux central, les composés de l'invention ont démontré des propriétés agonistes ou antagonistes 5-$HT_{1A}$. Ils peuvent donc être utiles dans le traitement de la migraine, de la dépression, de l'anxiété, de la schizophrénie, du stress et de la douleur.

La présente invention a plus particulièrement comme objet les dérivés de la napht-1-yl pipérazine de formule générale I :

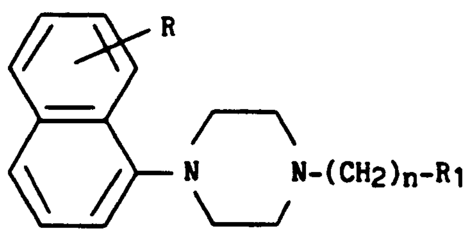

(I)

dans laquelle :

- n représente un nombre entier de 1 à 4,
- R représente un atome d'hydrogène, un atome d'halogène, un radical alkyle de 1 à 6 atomes de carbone, un radical hydroxy, ou un radical alcoxy de 1 à 6 atomes de carbone,
- $R_1$ représente un radical de formule $A_1$ :

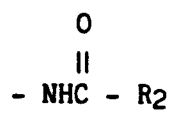

($A_1$)

(dans laquelle

- $R_2$ représente un radical alkyle de 1 à 6 atomes de carbone, un radical cycloalkyle de 3 à 7 atomes de carbone, un radical phényle -éventuellement substitué par un ou plusieurs atomes d'halogène, par un ou plusieurs radicaux alkyle de 1 à 6 atomes de carbone ou par un ou plusieurs radicaux alcoxy de 1 à 6 atomes de carbone-, un radical -O-alkyle de 1 à 6 atomes de carbone, un radical 3-pyridyle, un radical 2-pyrrolyle, un radical quinolyle, un radical 1-isoquinolyle, un radical 2-thiényle, un radical 3-indolyle)

  un radical de formule $A_2$ :

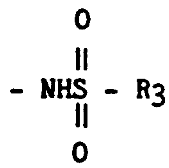

(A2)

(dans laquelle $R_3$ représente un radical alkyle de 1 à 6 atomes de carbone, un radical cycloalkyle de 3 à 7 atomes de carbone ou un radical phényle -éventuellement substitué par un ou plusieurs atomes d'halogène, par un ou plusieurs radicaux alkyle de 1 à 6 atomes de carbone ou par un ou plusieurs radicaux alcoxy de 1 à 6 atomes de carbone-),

un radical de formule $A_3$ :

$-NHCONHR_4$ **(A3)**

(dans laquelle $R_4$ représente un radical alkyle de 1 à 6 atomes de carbone ou un radical phényle éventuellement substitué par un ou plusieurs atomes d'halogène, par un ou plusieurs radicaux alkyle ou alcoxy de 1 à 6 atomes de carbone),

un radical o-sulfobenzoïque imido de formule $A_5$ :

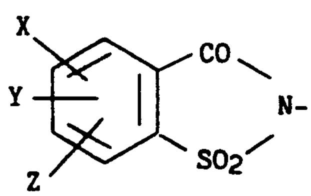

(A5)

ou un radical de formule $A_5$ :

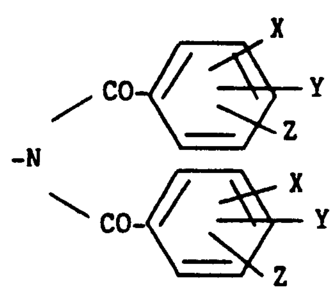

(A6)

(dans lesquelles X, Y et Z, identiques ou différents, représentent chacun un atome d'hydrogène, un atome d'halogène, un radical alkyle de 1 à 6 atomes de carbone ou un radical alcoxy de 1 à 6 atomes de carbone),

ou un radical de formule $A_7$ :

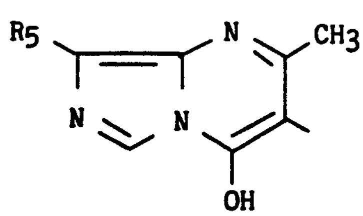

(A7)

(dans laquelle $R_5$ représente un radical carbamoyle, un radical cyano, un radical carboxy, ou un radical alcoxycarbonyle de 1 à 6 atomes de carbone),

ou un radical de formule $A_8$ :

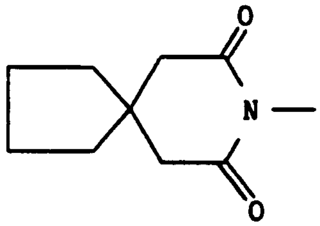

(A8)

leurs stéréoisomères et leurs sels d'addition à un acide minéral ou organique pharmaceutiquement acceptable.

La présente invention a également pour objet un procédé de préparation de composés de formule générale I, caractérisé en ce que l'on utilise comme matière première :

**a/ ou bien :**

un composé de formule II :

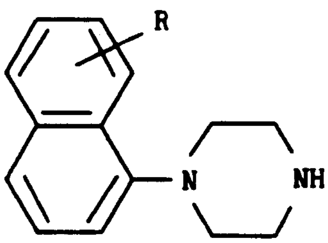

(II)

dans laquelle R a la même signification que pour la formule I,

que l'on condense :

**soit**

avec un nitrile de formule III :

$Hal(CH_2)_{n-1}CN$ **(III)**

dans laquelle Hal représente un atome d'halogène et n a la même signification que pour la formule I, dans un solvant organique, à température ambiante, en présence d'un sel d'un métal alcalin pour obtenir les composés de formule IV :

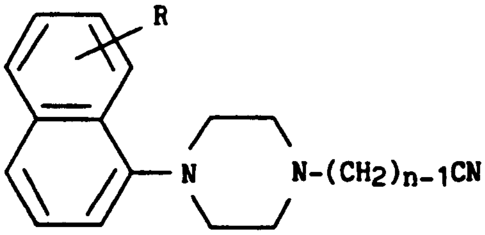

(IV)

dans laquelle R et n ont la même signification que pour la formule I, que l'on transforme à l'aide de l'hydrure de lithium et d'aluminium ou d'un autre réactif chimique équivalent,

à un composé de formule V :

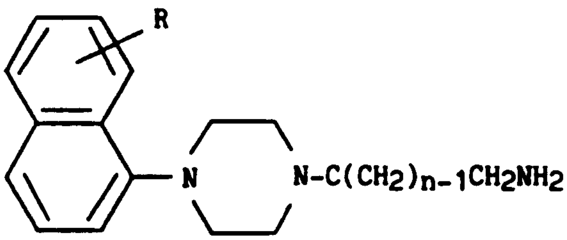

(V)

dans laquelle R et n ont la même signification que pour la formule I,
que l'on fait réagir

ou

avec une quantité équimolaire d'un composé de formule $VI_A$ ou $VI_B$ :

$ClCOR_2$ **($VI_A$)**

$ClSO_2R_3$ **($VI_B$)**

dans lesquelles $R_2$ et $R_3$ ont les mêmes significations que pour la formule I, pour obtenir respectivement les composés de formule I dans laquelle $R_1$ représente un radical de formule $A_1$ et les composés de formule I dans laquelle $R_1$ représente un radical de formule $A_2$,

composés de formule I dans laquelle $R_2$ représente un radical -O-alkyle, que l'on fait réagir, si on le souhaite, avec un composé de formule VII :

$R_4NH_2$ **(VII)**

dans laquelle $R_4$ a la même signification que pour la formule I,
pour former les composés de formule I dans laquelle $R_1$ représente un radical de formule $A_3$,

ou

avec un composé de formule VIII, en excès, :

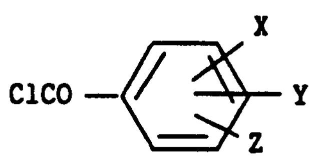

(VIII)

dans laquelle X, Y et Z ont la même signification que pour la formule I,
pour former un composé de formule I dans laquelle $R_1$ représente un radical de formule $A_6$,

**soit**

avec un composé de formule $IX_B$ :

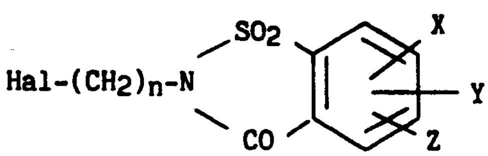

**($IX_B$)**

dans laquelle Hal a la même signification que pour la formule III et X, Y et Z ont la même signification que pour la formule I,

pour former les composés de formule I dans laquelle $R_1$ est un radical de formule $A_6$,

**soit**

avec un alcool de formule X :

$Hal(CH_2)_nOH$ **(X)**

dans laquelle n a la même signification que pour la formule I et la signification de Hal reste identique à celle donnée pour la formule III,

pour former les composés de formule XI :

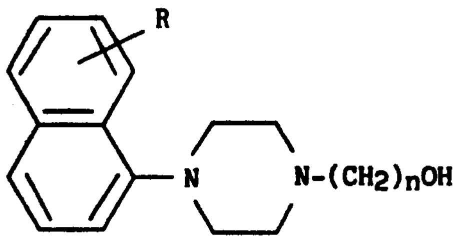

**(XI)**

dans laquelle R et n ont la même signification que pour la formule I,

que l'on soumet à l'action du chlorure de thionyle ou d'un autre réactif chimique équivalent pour former les composés de formule XII :

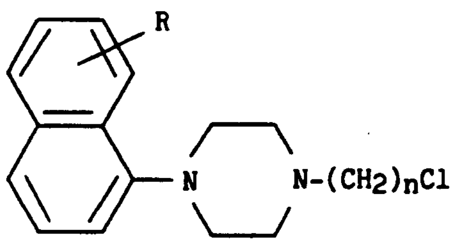

**(XII)**

dans laquelle R et n ont la même signification que pour la formule I,

que l'on condense

ou

avec l'acétoacétate d'éthyle pour former un composé de formule XIII,

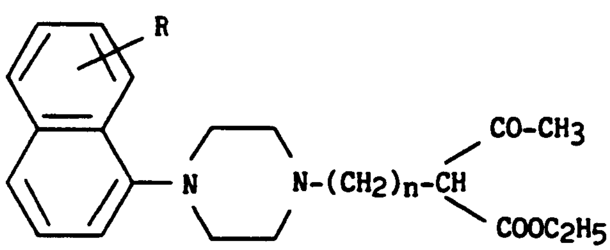

**(XIII)**

dans laquelle n et R ont la même signification que pour la formule I,

que l'on condense avec un dérivé de 4-amino imidazole de formule XIV :

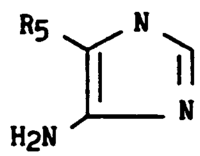

(XIV)

dans laquelle $R_5$ a la même signification que pour la formule I,

pour former les composés de formule I dans laquelle $R_1$ représente un radical de formule $A_7$,

ou

avec un composé de formule XV

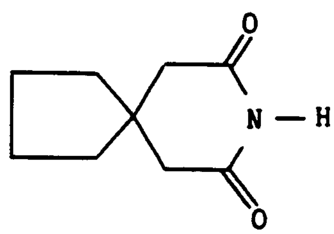

(XV)

pour former les composés de formule I dans laquelle $R_1$ représente un radical de formule $A_8$.

**b/ ou bien :**

un composé de formule XVI :

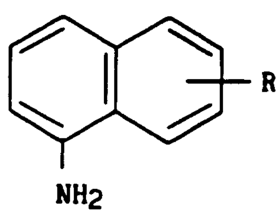

(XVI)

dans laquelle R a la même signification que dans la formule I,

que l'on condense avec de l'acide N-benzyliminodiacétique de formule XVII préalablement mis à reflux avec du carbonyldiimidazole dans un solvant anhydre comme le tétrahydrofurane selon la technique décrite par C.G. KRUSE et J.J. TROST (Recueil des travaux chimiques des Pays-Bas, 107, 303-309, 1988)

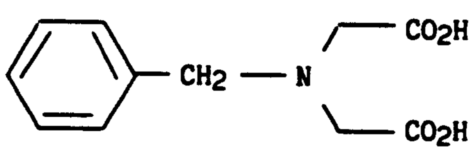

(XVII)

pour conduire à une pipérazine-2,6-dione de formule XVIII :

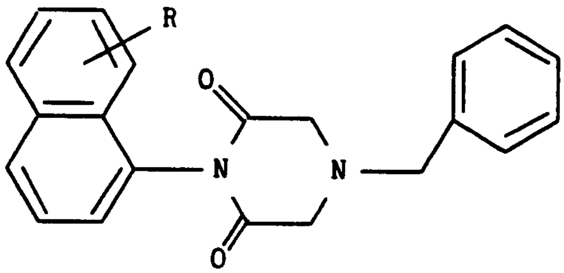

(XVIII)

dans laquelle R a la même signification que dans la formule I,

sur laquelle on réalise une hydrogénation catalytique en présence de charbon palladié comme catalyseur,

pour conduire à une pipérazine-2,6-dione de formule XIX :

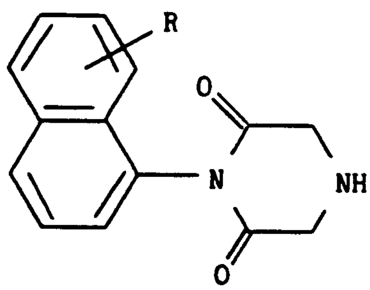

(XIX)

dans laquelle R a la même signification que dans la formule I,

que l'on condense

**soit**

avec un nitrile de formule III :

Hal - $(CH_2)_{n-1}$ - CN **(III)**

dans laquelle Hal et n ont la même signification que précédemment,

pour conduire à une pipérazine-2,6-dione de formule XX :

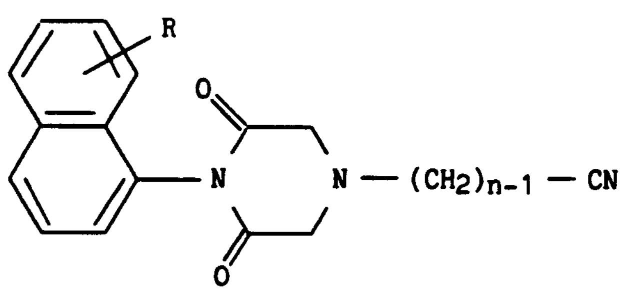

(XX)

dans laquelle R et n ont la même signification que dans la formule I,

que l'on réduit en présence du complexe borane-diméthylsulfure selon la technique décrite par H.C. BROWN et Coll. (J. Org. Chem., 47, 3153-3163, 1982)

pour conduire à une pipérazine de formule V :

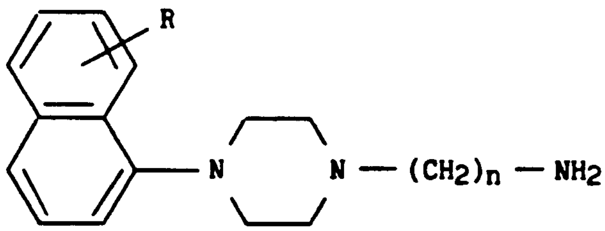

(V)

dans laquelle R et n ont la même signification que dans la formule I, que l'on fait réagir

ou

avec une quantité équimoléculaire d'un composé de formule $VI_A$ ou $VI_B$ :

**Cl CO $R_2$ ($VI_A$)**

**Cl $SO_2$ $R_3$ ($VI_B$)**

dans lesquelles $R_2$ et $R_3$ ont les mêmes significations que pour la formule I, pour obtenir respectivement les composés de formule I dans laquelle $R_1$ représente un radical de formule $A_1$, et les composés de formule I dans laquelle $R_1$ représente un radical de formule $A_2$,

composés de formule I dans laquelle $R_2$ représente un radical -O-alkyle, que l'on fait réagir, si on le souhaite, avec un composé de formule VII :

$R_4NH_2$ **(VII)**

dans laquelle $R_4$ a la même signification que pour la formule I,

pour former les composés de formule I dans laquelle $R_1$ représente un radical de formule $A_3$,

ou

avec un composé de formule VIII, en excès, :

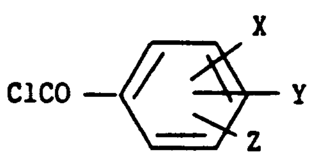

**(VIII)**

dans laquelle X, Y et Z ont la même signification que pour la formule I,

pour former un composé de formule I dans laquelle $R_1$ représente un radical de formule $A_6$,

**soit**

avec un alcool de formule X :

**Hal $(CH_2)_n$ OH (X)**

dans laquelle Hal et n ont la même signification que précédemment,

pour conduire à une pipérazine-2,6-dione de formule XXI :

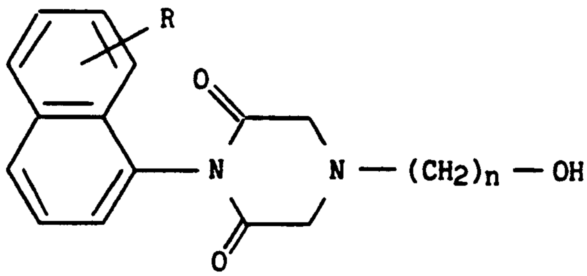

(XXI)

dans laquelle R et n ont la même signification que dans la formule I,

que l'on soumet à l'action de chlorure de thionyle ou d'un autre réactif chimique équivalent, pour conduire à une pipérazine-2,6-dione de formule XXII :

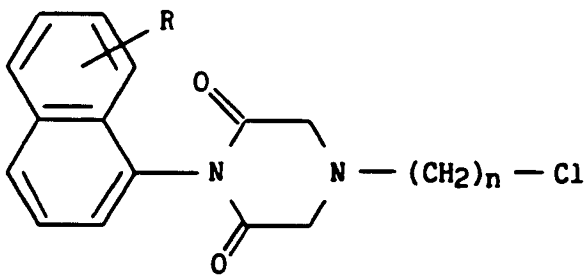

(XXII)

dans laquelle R et n ont la même signification que dans la formule I

que l'on réduit en présence du complexe borane-diméthylsulfure selon la technique décrite par H.C. BROWN et Coll. (J. Org. Chem., 47, 3153-3163, 1982)

pour conduire à une pipérazine de formule XII :

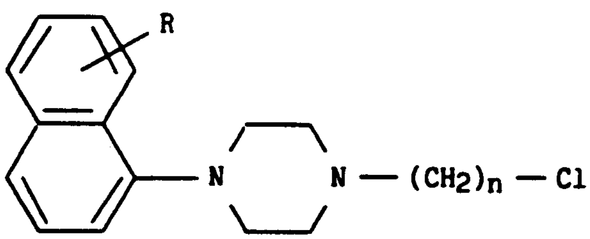

(XII)

dans laquelle R et n ont la même signification que dans la formule I,

que l'on condense

ou

avec un composé de formule $XXIII_B$ ou XV

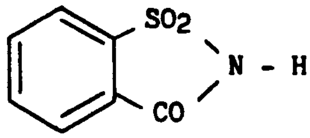

(XXIIIB)

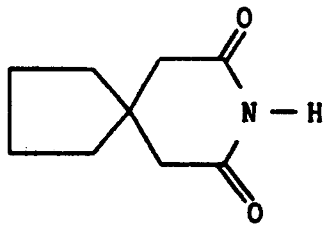

(XV)

pour conduire respectivement aux composés de formule I dans laquelle $R_1$ est un radical, $A_5$ ou $A_5$,

ou

avec l'acétoacétate d'éthyle pour former un composé de formule XIII

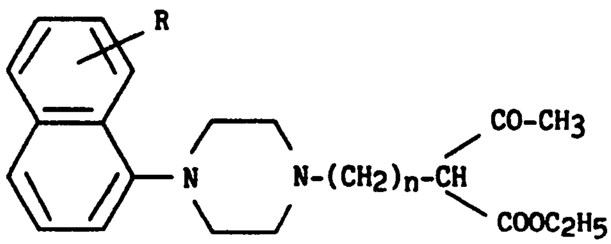

(XIII)

dans laquelle n et R ont la même signification que pour la formule I,
que l'on condense avec un dérivé de 4-amino imidazole de formule XIV :

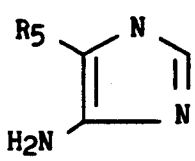

(XIV)

dans laquelle $R_5$ a la même signification que pour la formule I,
pour former les composés de formule I dans laquelle $R_1$ représente un radical de formule $A_7$,

**lesquels, composés de formule I, ensuite,**

si l'on désire, sont salifiés par un acide organique ou minéral, pharmaceutiquement acceptable, pour former les sels d'addition correspondants.

Les composés de formule II sont obtenus selon le procédé décrit dans Collection Czechoslov Chem.Commun.,(1975),40,p.1612. La préparation de la 1-(7-méthoxynapht-1-yl) pipérazine est aussi connue (J.Med.Chem., (1989),32,N°8,p.1921).

La réaction du composé de formule II avec le composé de formule III s'effectue à température ambiante, et de manière préférentielle en présence de carbonate de potassium ou de carbonate de sodium. Les composés de formule IV sont transformés en composé de formule V à température ambiante dans un solvant organique de préférence le tétrahydrofurane. Les mêmes conditions opératoires sont aussi utilisées pour la réaction des composés de formule V avec les composés de formule $VI_A$ ou $VI_B$. La réaction s'effectue en présence de triéthylamine, ainsi que la réaction des composés de formule V avec les

composés de formule VIII.

La réaction des composés de formule VII avec les composés de formule I dans laquelle $R_2$ représente un radical -O-alkyle est décrite dans J.Chem.Soc.,(1949),p.1732-1738.

Parmi les acides pharmaceutiquement acceptables pour la préparation des sels d'addition aux composés de formule générale I, on peut citer les acides chlorhydrique, phosphorique, fumarique, citrique, oxalique, sulfurique, ascorbique, tartrique, maléïque, mandélique, méthanesulfonique, etc....

Les composés de la présente invention possèdent des propriétés pharmacologiques fort intéressantes. Au niveau cardiovasculaire, les composés de l'invention diminuent la pression artérielle et la fréquence cardiaque chez le rat et chez le chien. Cette action résulte d'une inhibition centrale du tonus sympathique. En effet, les essais pharmacologiques ont démontré que la baisse de pression provoquée par l'administration i.v. des composés de l'invention chez le chien, s'accompagne d'une forte diminution de l'activité électrique du nerf sympathique rénal.

Cette diminution centrale du tonus sympathique résulte d'une activation des récepteurs $5HT_{1A}$ centraux au niveau du noyau rétrofacial (Eur.Journal of Pharm.,(1989),160,p.385-294). Les essais pharmacologiques ont aussi prouvé que les composés de l'invention sont environ 3 fois plus actifs que le flesinoxan, composé de référence ayant des propriétés antihypertensives, dues à son activité agoniste des récepteurs $5\text{-}HT_{1A}$ - (European Journal of Pharmacology,(1988),149,p.213-223). D'autre part, les composés de l'invention ont une activité bénéfique au niveau rénal (T.I.P.S.,(1989),10,p.469-471)

Les essais de binding ont confirmé que les composés de l'invention se comportent aussi comme de très puissants ligands des récepteurs $5\text{-}HT_{1A}$, avec une activité, agoniste ou antagoniste au niveau système nerveux central.

Les composés de l'invention trouvent donc leur application dans le traitement du stress (Neuropharmac.,(1989),Vol.25,N°5,p.471-476), de la migraine (T.I.P.S.,(1989),Vol.10,pp.200-204), de l'anxiété, de la dépression, de la schizophrénie et de la douleur (Pharmacology and Toxicology,(1989),64,p.3-5), (Drugs of the Future,(1988),13,N°5, p.429-437), (J.Neural.Transm.,(1988),74,p.195-198).

Les composés actifs au niveau des récepteurs $5\text{-}HT_{1A}$ peuvent aussi modifier le comportement alimentaire et sexuel (Jour. of Receptor Research, (1988),8,p.59-81).

L'invention s'étend aussi aux compositions pharmaceutiques renfermant comme principe actif au moins un composé de formule générale I, ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, suppositoires, solutions injectables ou buvables.

La posologie peut varier largement en fonction de l'âge, du poids du patient, de la nature et de la sévérité de l'affection ainsi que de la voie d'administration. D'une manière générale, la posologie unitaire s'échelonnera entre 0,05 et 10 mg et la posologie journalière, utilisable en thérapeutique humaine, entre 0,05 et 20 mg. La voie d'administration préférée est la voie orale ou parentérale.

Les exemples suivants, donnés à titre non-limitatif, illustrent l'invention.

Les points de fusion ont été mesurés selon la technique Micro-Köfler.

Les spectres de résonance magnétique nucléaire du proton (RMN-$^1$H) ou de carbone $^{13}$C (RMN-$^{13}$C) des composés de formule générale I ont été enregistrés selon le cas à 200 et 400 MHz et sont indiqués dans le Tableau I.

**EXEMPLE 1**

**Chlorhydrate de la 1-(6-méthoxynapht-1-yl) 4-[2-(4-fluorobenzoyl amino)éthyl] pipérazine**

Stade A

Chlorhydrate du 1-amino 6-méthoxy napthalène

19,5 g du 1-amino 6-hydroxy naphtalène sont ajoutés par petites quantités à une solution de méthanolate de sodium (2,82 g de sodium métal dans 80 ml de méthanol). Le milieu est agité une heure à température ambiante puis, le solvant évaporé sous vide. Le résidu obtenu est dissout dans 700 ml d'acétone et 9,27 g de sulfate de diméthyle sont ajoutés goutte à goutte tout en maintenant une température inférieure à 60°C. L'addition terminée, le milieu est agité 12 heures à température ambiante. Le précipité formé est filtré, le filtrat est traité au charbon actif et le solvant évaporé sous vide. L'huile ainsi obtenue est purifiée par chromatographie sur 1 kg de silice 70-230 Mesh en utilisant comme éluant le

dichlorométhane.
Rendement : 55%
Spectre de résonance magnétique nucléaire du proton (Solvant $CDCl_3$): 3,9 ppm,s,3H; 4,1 ppm,1H échangeable, 6,6 ppm,dd,1H; 7,0-7,3 ppm,m,3H; 7,05 ppm,m,1H; 7,65 ppm,d,1H.

Stade B

Chlorhydrate de la 1-(6-méthoxynapht-1-yl) pipérazine

Un mélange de 4,1 g du composé obtenu au stade précédent et de 4,7 g de chlorhydrate de N,N-bis-(chloroéthyl)amine dans 150 ml de chlorobenzène est porté au reflux pendant 24 heures. Le précipité est filtré, lavé à l'éther et recristallisé dans l'éthanol pour fournir le produit attendu.
Rendement : 70%.
Spectre de résonance magnétique nucléaire du proton (Solvant DMSO-$d_6$): 3,1-3,5 ppm,m,8H; 3,9 ppm,s,3H; 6,70 ppm,d,1H; 7,15 ppm,m,1H; 7,50 ppm,d,1H; 7,8-7,5 ppm,m,3H.

Stade C

[4-(6-méthoxynapht-1-yl) pipérazino] acétonitrile

Un mélange de 5,8 g du composé obtenu dans le Stade B, de 2,8 g de 2-bromoacétonitrile et de 6,4 g de carbonate de potassium dans 250 ml d'acétone est agité à température ambiante jusqu'à complète disparition de la pipérazine de départ constatée par CCM (éluant : Dichlorométhane Acétone 95:5 V/V). Le milieu est alors filtré et le solvant évaporé sous vide. Le produit cristallise dans un minimum d'éther.
Rendement : 70%

**Analyse élémentaire :**

| | C% | H% | N% |
|---|---|---|---|
| Calculé : | 72,57 | 6,81 | 14,93 |
| Trouvé : | 72,44 | 7,01 | 14,67 |

Stade D

1-(6-méthoxynapht-1-yl) 4-(2-aminoéthyl) pipérazine

Une solution contenant 4,2 g du composé obtenu dans le Stade C, dans 25 ml de tétrahydrofurane est ajoutée goutte à goutte à température ambiante à une suspension de 1,1 g d'hydrure de lithium et d'aluminium dans 25 ml de tétrahydrofurane agitée sous atmosphère d'azote. L'agitation est maintenue 20 minutes après la fin de l'addition, puis le milieu réactionnel est hydrolysé avec la quantité nécessaire d'eau saturée au chlorure d'ammonium. Le précipité formé est filtré, lavé au tétrahydrofurane, et les filtrats sont concentrés sous vide pour fournir une huile.
Rendement : 74%
Spectre de résonance magnétique nucléaire du proton (Solvant $CDCl_3$): 3,1-3,6 ppm,m,4H; 3,1-4,0 ppm,m + m,4 + 4H; 3,85 ppm,s,3H; 7,0 ppm,d,1H; 7,15 ppm,dd,1H; 7,2-7,4 ppm,t + d,1 + 1H; 7,6 ppm,d,1H; 7,95 ppm,d,1H.

Stade E

A une solution agitée à température ambiante de 3 g du composé obtenu au Stade D et de 1,7 g de triéthylamine dans 20 ml de tétrahydrofurane, on ajoute 1,8 g de chlorure de 4-fluoro benzoyle dans 50 ml de tétrahydrofurane. Après deux heures de contact, le milieu réactionnel est filtré. Le filtrat concentré sous vide et le produit obtenu est cristallisé dans l'éther.
Rendement : 76%
Point de Fusion : 180 °C.

Les 3,1 g de la base sont dissouts dans 300 ml d'éthanol et une quantité nécessaire de méthanol chlorhydrique 6N est ajoutée goutte à goutte. Le chlorhydrate précipité.
Point de Fusion : ≧ 260 ° C.

**EXEMPLE 2**

**Chlorhydrate de la 1-(6-hydroxynapht-1-yl) 4-[2-(4-fluorobenzoyl amino)éthyl]pipérazine**

Une solution de 0,85 g du composé de l'exemple 1 sous forme de base dans 40 ml de dichlorométhane est refroidie à -20 ° C sous atomosphère d'azote. 10 ml d'une solution molaire de tribromure de bore dans le dichlorométhane sont alors ajoutés goutte à goutte tout en maintenant la température à -20 ° C. L'addition terminée, le milieu est lentement réchauffé jusqu'à la température ambiante et agité à cette température une heure avant d'être hydrolysé par 2 ml d'une solution ammoniacale. La phase organique décantée est séchée sur sulfate de magnésium anhydre et le solvant évaporé sous vide ; le résidu est dissout dans 20 ml d'éthanol et le chlorhydrate est précipité par addition de 0,4 ml d'éthanol chlorhydrique 6N.
Rendement : 72%
Point de Fusion : 216 ° C.

**EXEMPLE 3**

**Chlorhydrate de la 1-(7-méthoxynapht-1-yl) 4-[2-(4-fluorobenzoyl amino)éthyl] pipérazine**

Stade A

[4-(7-méthoxynapht-1-yl) pipérazino] acétonitrile

Ce composé est obtenu à partir de la (7-méthoxynapht-1-yl) pipérazine selon le procédé décrit dans l'exemple 1, Stade C.
Rendement : 74%
Spectre de résonance magnétique nucléaire du proton (Solvant $CDCl_3$): 2,9 ppm,t,4H; 3,2 ppm,m,4H; 3,65 ppm,s,2H; 3,95 ppm,s,3H; 7,0-7,35 ppm,m,3H; 7,5 ppm,m,2H; 7,7 ppm,d,1H.

Stade B

1-(7-méthoxynapth-1-yl) 4-(2-aminoéthyl) pipérazine

Ce composé est obtenu selon la méthode décrite à l'exemple 1, Stade D, à partir du [4-(7-méthoxynapht-1-yl) pipérazino] acétonitrile.
Rendement : 97%
Spectre de résonance magnétique nucléaire du proton (Solvant DMSO-$d_6$): 3,1-3,55 ppm,m,10H; 3,75 ppm,d,2H; 3,95 ppm,s,3H; 7,0-7,4 ppm,m,3H; 7,4 ppm,d,1H; 7,6 ppm,d,1H; 7,85 ppm,d,1H; 8,6 ppm,1H échangeable, 11,5 ppm,1H échangeable.

Stade C

Le chlorhydrate de la 1-(7-méthoxynapht-1-yl) 4-[2-(4-fluorobenzoyl) aminoéthyl] pipérazine a été obtenu à partir du composé du stade précédent selon le procédé décrit dans l'exemple 1, Stade E.
Rendement : 40%
Point de Fusion : 221 ° C.

**EXEMPLE 4**

**Chlorhydrate de la 1-(7-méthoxynapht-1-yl) 4-[2-(bis(4-fluorobenzoyl)amino)éthyl] pipérazine**

Pour obtenir ce composé, la phase éthérée issue lors de la cristallisation du composé de l'exemple 3 sous forme de base est concentrée et chromatographiée sur 20 g de silice 70-230 Mesh en utilisant comme éluant un mélange de dichlorométhane et d'acétone (95:5 V/V).

Rendement : 20%

La base ensuite est dissoute dans l'éther isopropylique et transformée en chlorhydrate avec une solution d'acide chlorhydrique dans l'éther éthylique.

Point de Fusion : 234 ° C.

**EXEMPLE 5**

**Chlorhydrate de la 1-(7-hydroxynapht-1-yl) 4-[2-(4-fuorobenzoyl amino)éthyl] pipérazine**

Ce composé a été préparé selon le procédé décrit dans l'exemple 2 à partir du composé de l'exemple 3.

Rendement : 80%

Point de Fusion : 230 ° C

**EXEMPLE 6**

**Chlorhydrate de la 1-(7-méthoxynapht-1-yl) 4-(2-tosylaminoéthyl pipérazine**

Ce composé a été préparé selon la méthode décrite dans l'exemple 1, Stade E, à partir de la 1-(7-méthoxynapth-1-yl) 4-(2-aminoéthyl) pipérazine et du chlorure de tosyle en solution dans le chloroforme.

Rendement : 44%

Point de fusion : 277 ° C

**EXEMPLE 7**

**Chlorhydrate de la 1-(7-méthoxynapht-1-yl) 4-[2-(éthoxycarbonyl amino)éthyl] pipérazine**

Ce composé a été préparé comme il est indiqué dans l'exemple précédent à partir de la 1-(7-méthoxynapth-1-yl) 4-(2-aminoéthyl) pipérazine et du chloroformiate d'éthyle dans le chloroforme.

Rendement : 55%

Point de Fusion : 208 ° C.

**EXEMPLE 8**

**Chlorhydrate de la 1-(7-méthoxynapht-1-yl) 4-(2-nicotinoyl aminoéthyl) pipérazine**

Ce composé a été préparé selon le procédé décrit dans l'exemple 1, Stade E, à partir de la 1-(7-méthoxynapth-1-yl) 4-(2-aminoéthyl) pipérazine et du chlorure de l'acide nicotinique.

Rendement : 70%

Point de Fusion : 204 ° C.

**EXEMPLE 9**

**Chlorhydrate de la 1-(7-méthoxynapht-1-yl) 4-[4-(4-fluorobenzoylamino)but-1-yl] pipérazine**

Stade A

4-[4-(7-méthoxynapht-1-yl) pipérazino] butyronitrile

Ce composé a été préparé selon le procédé décrit dans l'exemple 1, Stade C, à partir de la 4-(7-méthoxynapth-1-yl) pipérazine et du 4-bromo-butyronitrile.

Rendement : 98%

Spectre de résonance magnétique nucléaire du proton (Solvant $CDCl_3$): 1,85 ppm,m,2H; 2,5 ppm,t,2H; 2,6 ppm,t,2H; 2,5-2,9 ppm,m,4H; 3,1 ppm,m,4H; 3,9 ppm,s,3H; 7,10 ppm,d,1H; 7,15 ppm,dd,1H; 7,25 ppm,t,1H; 7,5 ppm,d,1H; 7,55 ppm,s,1H; 7,7 ppm,d,1H.

Stade B

4-(7-méthoxynapht-1-yl) 1-(4-aminobut-1-yl) pipérazine

Ce composé a été préparé à partir du composé décrit au stade précédent selon le procédé décrit dans l'exemple 1, Stade D.
Rendement : 80%

Stade C

Le composé attendu a été préparé à partir de la 1-(7-méthoxynapth-1-yl) 4-(4-aminobut-1-yl) pipérazine et du chlorure de l'acide 4-fluorobenzoïque.
Rendement : 60%
Point de Fusion : 224 ° C.

**EXEMPLE 10**

**Chlorhydrate de 8-carbamoyl 4-hydroxy 3-{2-[4-(7-méthoxynapht-1-yl) pipérazino] éthyl} 2-méthyl imidazo (1,5-a) pyrimidine**

Stade A

Chorhydrate de 2-[4-(7-méthoxynapht-1-yl) pipérazino] éthanol

Un mélange de 5 g de (7-méthoxynapht-1-yl) pipérazine de 3,4 g de 2-iodoéthanol et de 5,5 g de carbonate de potassium dans 75 ml d'acétonitrile est porté au reflux 24 heures. Le milieu est ensuite filtré, et le solvant est évaporé. Le résidu est purifié par chromatographie sur 150 g de silice 70-230 Mesh en utilisant comme éluant un mélange de dichlorométhane, de méthanol et d'ammoniaque (95:4,5:0,5 V/V). Les fractions contenant le produit sont évaporées et le résidu est dissout dans 20 ml d'éthanol, puis 2,1 ml d'éthanol chlorhydrique 6N sont ajoutés goutte à goutte. Le chlorhydrate attendu est obtenu après précipitation par l'éther.
Rendement : 70%
Spectre de résonance magnétique nucléaire du proton (Solvant DMSO-$d_6$): 3,1-3,8 ppm,m + m,2H + 8H; 3,8-4,0 ppm,s + m,3H + 2H; 7,1-7,25 ppm,dd + d,1H + 1H; 7,3 ppm,t,1H; 7,4 ppm,d,1H; 7,6 ppm,d,1H; 7,85 ppm,d,1H; 10,7-11,1 ppm,massif échangeable.

Stade B

1-(7-méthoxynapht-1-yl) 4-(2-chloroéthyl) pipérazine

3,6 g de chlorure de thionyle sont ajoutés goutte à goutte à une suspension contenant 3,5 g du composé obtenu au stade précédent dans 100 ml de chloroforme. L'addition terminée, le milieu est chauffé 1 heure 30 min à reflux. Ensuite, le précipité est filtré et lavé à chaud dans l'acétone.
Rendement : 68%
Point de Fusion : 176 ° C.
Spectre de résonance magnétique nucléaire du proton (Solvant DMSO-$d_6$): 3,1-3,8 ppm,m,10H; 3,9 ppm,s,3H; 4,15 ppm,t,2H; 7,0-7,4 ppm,d + t + dd + d, 1H + 1H + 1H + 1H; 7,6 ppm,d,1H; 7,85 ppm,d,2H; 11,3-11,7 ppm,massif échangeable.

Stade C

Chlorhydrate de 2-acétyl 4-[4-(7-méthoxynapht-1-yl) pipérazino] butyrate d'éthyle

A 0 ° C, ajouter 0,182 mole d'acétoacétate d'éthyle à une suspension contenant 0,182 mole d'hydrure de sodium dans 800 ml de tétrahydrofurane. Maintenir le milieu réactionnel une heure à 20 ° C puis ajouter 0,182 mole d'iodure de sodium. Refroidir à 0 ° C et additionner 0,182 mole du composé obtenu au stade précédent. Porter à reflux pendant 12 heures et ensuite concentrer sous vide. Reprendre le résidu dans l'eau et extraire au dichlorométhane. Purifier l'huile obtenue par chromatographie sur colonne de silice de

70-230 Mesh en éluant avec un mélange de dichlorométhane et d'acétone (95:5 V/V).
Rendement : 50%

Le 2-acétyl 4-[4-(7-méthoxynapht-1-yl) pipérazino] butyrate d'éthyle obtenu est ensuite dilué dans l'éther et le chlorhydrate est précipité après addition de la quantité nécessaire d'éther chlorhydrique.

Spectre de résonance magnétique nucléaire du proton (solvant $CDCl_3$): 1,3 ppm,t,3H; 2,25-2,6 ppm,m + s,2H + 3H; 3,0-4,1 ppm,s + m + m + m, 3H + 4H + 6H + 1H; 4,2 ppm,q,2H; 7,1-7,35 ppm,m,3H; 7,4 ppm,s,1H; 7,6 ppm,dd,1H; 7,8 ppm,d,1H; 12,3-13,3 ppm,1H échangeable.

Stade D

Mélanger 0,01 mole de chlorhydrate de 4-amino 5-carbamoyl imidazole, 0,011 mole de l'ester préparé dans l'étape précédente et 10,5 g d'acide phosphorique. Porter à 80 ° C pendant 30 minutes.

Hydrolyser par de la glace et neutraliser avec de la soude concentrée pour obtenir la précipitation de la 8-carbamoyl 4-hydroxy 3-{2-[4-(7-méthoxynapht-1-yl) pipérazino] éthyl} 2-méthyl imidazo [1,5-a] pyrimidine. Salifier ensuite avec le méthanol chlorhydrique.
Rendement : 42%
Point de Fusion : 220 ° C

**EXEMPLE 11**

**Chlorhydrate de 8-carbomoyl 4-hydroxy 2-méthyl 3-[2-(4-napht-1-yl pipérazino) éthyl] imidazo [1,5-a] pyrimidine**

Stade A

Chlorhydrate de 2-(4-napht-1-yl pipérazino) éthanol

Ce composé a été préparé à partir du 2-iodoéthanol et de la napht-1-yl pipérazine selon le procédé décrit dans l'exemple 10, Stade A.
Rendement : 85%

Stade B

1 napht-1-yl 4-(2-chloroéthyl) pipérazine

Ce composé a été préparé à partir du produit obtenu au stade précédent et selon le procédé décrit dans l'exemple 10, Stade B.
Rendement : 50%
Spectre de résonance magnétique nucléaire du proton (Solvant $CDCl_3$): 2,6-3,0 ppm,t + m,2H + 4H; 3,1 ppm,m,4H; 3,7 ppm,t,2H; 7,1 ppm,dd,1H; 7,6-7,3 ppm,m,4H; 8,2 ppm,m,1H; 8,4 ppm,m,1H.

Stade C

Chlorhydrate de 2-acétyl 4-[4-(napht-1-yl pipérazino)] butyrate d'éthyle

Ce composé a été préparé selon le procédé décrit dans l'exemple 10, Stade C, à partir de l'acétoacétate d'éthyle et de la 1-napht-1-yl 4-(2-chloroéthyl) pipérazine.
Rendement : 40%
Spectre de résonance magnétique nucléaire du proton (Solvant $CDCl_3$): 1,3 ppm,t,3H; 2,0-2,3 ppm,m,2H; 2,3 ppm,s,3H; 2,45 ppm,t,2H; 2,65 ppm,m,4H, 3,1 ppm,m,4H; 3,6 ppm,t,1H; 4,2 ppm,q,2H; 7,05 ppm,dd,1H; 7,3-7,6 ppm, m,4H; 7,8 ppm,m,1H; 8,15 ppm,m,1H.

Stade D

Le chlorhydrate de 8-carbamoyl 4-hydroxy 2-méthyl 3-[2-(4-napht-1-ylpipérazino) éthyl] imidazo [1,5-a] pyrimidine a été préparé à partir de l'ester obtenu au stade précédent et du 4-amino 5-carbamoyl imidazole selon le procédé décrit dans l'exemple 10, Stade D.
Rendement : 30%

Point de Fusion : > 260 ° C

**EXEMPLE 12**

**Chlorhydrate de la 1-(7-méthoxynapht-1-yl) 4-(2-phtalimido éthyl) pipérazine**

Porter au reflux un mélange de 1,5 g de (7-méthoxynapht-1-yl) pipérazine et de 1,5 g de 2-bromoéthyl phtalimide dans 100 ml d'acétone en présence de 1,6 g de carbonate de potassium. Agiter durant 24 heures. Refroidir. Concentrer. Triturer l'huile obtenue dans l'éther. Ajouter la quantité nécessaire d'éther chlorhydrique pour obtenir le chlorhydrate attendu.
Rendement : 63%
Point de Fusion : 252 ° C

**EXEMPLE 13**

**Chlorhydrate de la 1-(7-méthoxynapht-1-yl)-4-[2-(thien-2-oyl amino) éthyl] pipérazine**

Ce composé a été préparé selon le procédé décrit dans l'exemple 1, Stade E, à partir de la 1-(7-méthoxynapht-1-yl) 4-(2-aminoéthyl) pipérazine et du chlorure de thiophène-2-carbonyle.
Rendement : 81%
Point de Fusion : 237 ° C

**EXEMPLE 14**

**Chlorhydrate de la 1-(7-méthoxynapht-1-yl)-4-[2-(4-fluorobenzoylamino) éthyl] pipérazine**

Stade A

1-(7-méthoxynapht-1-yl)-4-benzylpipérazine-2,6-dione

A une suspension contenant 58 mmoles (1 équivalent) d'acide N-benzyliminodiacétique dans 200 ml de tétrahydrofurane anhydre, on ajoute 126 mmoles (2,2 équivalents) de carbonyldiimidazole. L'ensemble est porté à reflux jusqu'à la fin du dégagement de $CO_2$. Une solution contenant 58 mmoles (1 équivalent) de 1-amino-7-méthoxynaphtalène dans 40 ml de tétrahydrofurane anhydre est alors ajoutée au mélange précédent. L'ensemble est porté 20 heures à reflux, le solvant est évaporé et le résidu obtenu repris par 300 ml d'éthanol anhydre. Le précipité formé est filtré, puis dissous dans 200 ml de dichlorométhane. L'insoluble est filtré et le filtrat concentré. Le produit attendu est alors obtenu après reprise dans l'éther isopropylique puis filtration.
Rendement : 82%
Point de fusion : 178 ° C

Stade B

1-(7-méthoxynapht-1-yl)-pipérazine-2,6-dione

Une suspension contenant 43 mmoles du produit obtenu au Stade précédent agitée en présence de 1 g de Palladium sur charbon à 10 % dans 500 ml de méthanol est hydrogénée à pression atmosphérique et à température ambiante pendant 3 h 30. Après filtration du catalyseur et évaporation du solvant, on obtient le produit attendu.
Rendement : 97%
Point de fusion : 232 ° C

Stade C

1-(7-méthoxynapht-1-yl)-4- (cyanométhyl) pipérazine-2,6-dione

11 mmoles du produit obtenu au Stade précédent sont agitées à température ambiante avec 17 mmoles de bromoacétonitrile et 13 mmoles de triéthylamine dans 60 ml d'un mélange acétone anhydre/diméthylformamide anhydre (50/50). Après hydrolyse par 200 ml d'eau, le produit attendu est obtenu après filtration du précipité.
Rendement : 64%
Point de fusion : 204 ° C

Stade D

1-(7-méthoxynapht-1-yl)-4-(2-aminoéthyl) pipérazine

A une solution contenant 6,5 mmoles (1 équivalent) du produit obtenu au Stade précédent dans 70 ml de tétrahydrofurane anhydre à 60 ° C, on ajoute lentement 13 ml d'une solution 2M (4 équivalents) de complexe borane-diméthylsulfure. L'ensemble est porté au reflux 45 minutes, tout en distillant un mélange diméthylsulfure-tétrahydrofurane. Le volume réactionnel est maintenu constant lors de cette distillation par addition de tétrahydrofurane. Après hydrolyse, à température ambiante par 13,4 ml d'une solution d'acide chlorhydrique 6N, le mélange est porté au reflux 30 minutes.

Le produit attendu est obtenu après addition de 54 ml de soude 2N, par extraction avec 3 fois 100 ml de dichlorométhane, puis chromatographie sur gel de silice en utilisant comme éluant un mélange dichlorométhane/méthanol/ammoniaque (90/10/1)
Rendement : 50%

Stade E

chlorhydrate de la 1-(7-méthoxynapht-1-yl)-4-[2-(4-fluorobenzoylamino)éthyl] pipérazine

Le produit attendu est obtenu comme décrit au Stade C de l'exemple 3.

**EXEMPLE 15**

**Chlorhydrate de la 1-(7-méthoxynapht-1-yl)-4-(2-butyrylamino-éthyl) pipérazine**

En procédant comme dans l'exemple 14 mais en remplaçant au Stade E le chlorure de 4-fluorobenzoyle par le chlorure de butyryle, on obtient le produit attendu.
Rendement : 91%
Point de fusion : 190 ° C

Microanalyse élémentaire :

| | C % | H % | N % | Cl % |
|---|---|---|---|---|
| calculé | 64,35 | 7,72 | 10,72 | 9,05 |
| trouvé | 64,10 | 7,82 | 10,74 | 9,07 |

**EXEMPLE 16**

**Chlorhydrate de la 1-(7-méthoxynapht-1-yl)-4-(2-cyclopropylcarbonyl 2-cyclopropylcarbonyl aminoéthyl) pipérazine**

En procédant comme dans l'exemple 14 mais en remplaçant au Stade E le chlorure de 4-fluorobenzoyle par le chlorure de cyclopropylcarbonyl, on obtient le produit attendu.
Point de fusion : 204 ° C

Microanalyse élémentaire :

| | C % | H % | N % | Cl % |
|---|---|---|---|---|
| calculé | 64,69 | 7,24 | 10,78 | 9,09 |
| trouvé | 64,48 | 7,58 | 10,68 | 8,86 |

**EXEMPLE 17**

**Chlorhydrate de la 1-(7-méthoxynapht-1-yl)-4-[2-(4-fluorophénylsulfonamido) éthyl] pipérazine**

En procédant comme dans l'exemple 14 mais en remplaçant au Stade E le chlorure de 4-fluorobenzoyle par le chlorure de 4-fluorobenzènesulfonyl, on obtient le produit attendu.
Rendement : 79%
Point de fusion : 229 °C

Microanalyse élémentaire :

| | C % | H % | N % | Cl % | S % |
|---|---|---|---|---|---|
| calculé | 57,55 | 5,67 | 8,75 | 7,39 | 6,68 |
| trouvé | 57,96 | 5,98 | 8,68 | 7,21 | 6,66 |

**EXEMPLE 18**

**Chlorohydrate de la 1-(7-méthoxynapht-1-yl)-4-[2-(indol-3-yl-carboxamido) éthyl] pipérazine**

En procédant comme dans l'exemple 14 mais en remplaçant au Stade E le chlorure de 4-fluorobenzoyle par le chlorure de l'acide 3-indolecarboxylique, on obtient le produit attendu.
Rendement : 67%
Point de fusion : 242 °C

Microanalyse élémentaire :

| | C % | H % | N % | Cl % |
|---|---|---|---|---|
| calculé | 67,16 | 6,29 | 12,05 | 7,62 |
| trouvé | 67,04 | 6,45 | 11,96 | 7,72 |

**EXEMPLE 19**

**Chlorhydrate de la 1-(7-méthoxynapht-1-yl)-4-[2-(8-aza-7,9 dioxospiro(4,5) décan-8-yl) éthyl] pipérazine**

Les Stades A et B sont identiques à ceux décrits dans l'exemple 10.

Stade C

Une solution contenant 0,49 g de 8-aza-7,9-dioxospiro (4,5) décane dans 10 ml de diméthylformamide est lentement additionnée à une suspension contenant 0,13 g d'hydrure de sodium à 60 % dans 30 ml de diméthylformamide.

L'ensemble est porté 30 minutes à 70 °C. Après refroidissement, une solution contenant 1 g du produit

obtenu au stade précédent est additionnée au mélange et l'ensemble est porté au reflux 12 heures.

Le produit attendu est obtenu après évaporation, purification par chromatographie sur silice en utilisant comme solvant d'élution un mélange dichlorométhane/acétone (90/10) et précipitation du chlorhydrate dans de l'éther chlorhydrique.

Rendement : 60%

Point de fusion : 216 °C

**Microanalyse élémentaire :**

| | C % | H % | N % | Cl % |
|---|---|---|---|---|
| calculé | 63,30 | 7,06 | 8,51 | 11,48 |
| trouvé | 63,66 | 7,06 | 8,27 | 11,74 |

TABLEAU I

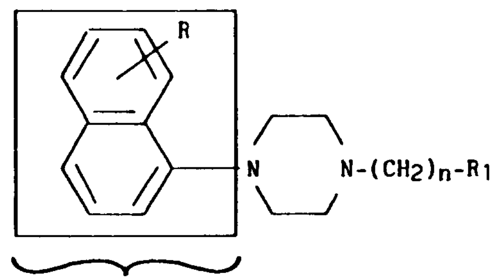

NAPH

| EX. N° | NAPH | n | $R_1$ | Spectre RMN (Solvant) s = sel b = base |
|---|---|---|---|---|
| 1 | 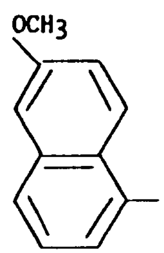 | 2 | 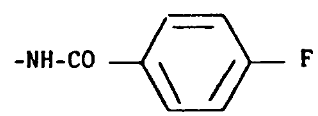 | (DMSO-d6) s RMN-$^1$H<br>3,1-4,0 ppm,m+m,4+8H; 3,85 ppm,s,3H; 7,0 ppm,d,1H; 7,15 ppm,dd,1H; 7,2-7,5 ppm,t+t+d,1+2+1H; 7,6 ppm,d,1H; 7,95-8,1 ppm,dd+d,2H+1H; 9,1 ppm,1H échangeable; 10,8-11,2 ppm,1H échangeable |

TABLEAU I
SUITE 1

| EX. N° | NAPH | n | $R_1$ | Spectre RMN (Solvant) s = sel b = base |
|---|---|---|---|---|
| 2 | 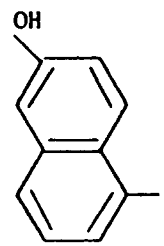 | 2 | 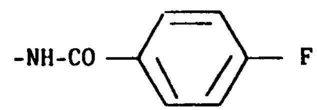 | (DMSO-$d_6$) s RMN-$^1$H 3,0-3,6 ppm,m,8H; 3,6-3,85 ppm,m,4H; 6,9 ppm,d,1H; 7,0-7,2 ppm,m,2H; 7,2-7,5 ppm,t+d+t,1+1+2H; 7,9-8,1 ppm,d,1H; 9,0 ppm,1H échangeable; 9,8 ppm,1H échangeable; 10,7 ppm,1H échangeable |
| 3 | 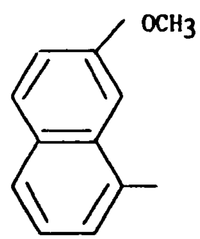 | 2 | 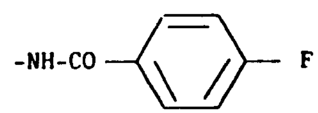 | (DMSO-$d_6$) s RMN-$^1$H 3,1-3,6 ppm,m,8H; 3,6-3,85 ppm,m,4H; 3,9 ppm,s,3H; 7,1-7,45 ppm,m,6H; 7,6 ppm,d, 1H; 7,85 ppm,d,1H; 8,05 ppm,dd,2H; 9,0 ppm, 1H échangeable; 10,95 ppm,1H échangeable |
| 4 | 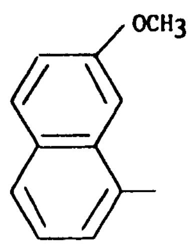 | 2 | 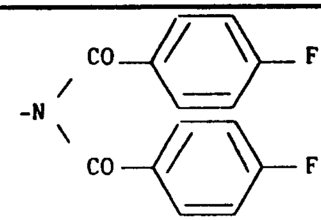 | (DMSO-$d_6$) s RMN-$^1$H 3,1-3,85 ppm,m,10H; 3,9 ppm,s,3H; 4,45 ppm,m,2H; 7,0-7,2 ppm,dd+d+t,1+1+4H; 7,3 ppm,t,1H; 7,4 ppm,d,1H; 7,65 ppm,d+dd, 1+4H; 7,85 ppm,d,1H; 10,9-11,2 ppm,1H échangeable |

TABLEAU I
SUITE 2

| EX. N° | NAPH | n | $R_1$ | Spectre RMN (Solvant) <u>s</u> = sel <u>b</u> = base |
|---|---|---|---|---|
| 5 | 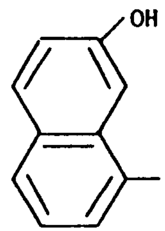 | 2 | 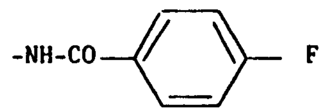 | (DMSO-$d_6$) <u>s</u> RMN-$^1H$ 3,2-3,55 ppm,<u>m</u>,8H; 3,8 ppm,<u>m</u>,4H; 7,05 ppm,<u>dd</u>,2H; 7,1-7,4 ppm,<u>m</u>,4H; 7,5 ppm,<u>d</u>, 1H; 7,75 ppm,<u>d</u>,1H; 8,1 ppm,<u>dd</u>,2H |
| 6 | 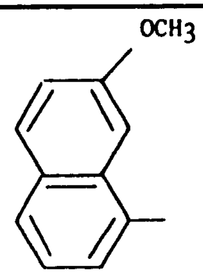 | 2 | 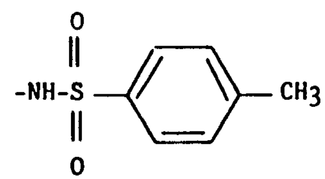 | (DMSO-$d_6$) <u>s</u> RMN-$^1H$ 2,4 ppm,<u>s</u>,3H; 3,1-3,8 ppm,<u>m</u>,12H; 3,9 ppm, <u>s</u>,3H; 7,15 ppm,<u>d</u>,1H; 7,20 ppm,<u>dd</u>,1H; 7,30 ppm, <u>t</u>,1H; 7,40 ppm,<u>d</u>,1H; 7,45 ppm,<u>d</u>,2H; 7,60 ppm,<u>d</u>,1H; 7,75 ppm,<u>d</u>,2H; 7,8 ppm,<u>d</u>,1H; 8,1 ppm,1H échangeable; 10,9-11,2 ppm,1H échangeable |

TABLEAU I

SUITE 3

| EX. N° | NAPH | n | $R_1$ | Spectre RMN (Solvant) <u>s</u> = sel <u>b</u> = base |
|---|---|---|---|---|
| 7 | 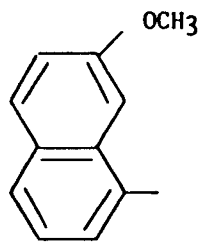 | 2 | $-NH-COOC_2H_5$ | ($DMSO-d_6$) <u>s</u> RMN-$^1H$ 1,2 ppm,<u>t</u>,3H; 3,1-3,75 ppm,<u>m</u>,10H; 3,7-4,0 ppm,<u>s</u>+<u>m</u>,3+2H; 4,05 ppm,<u>q</u>,2H; 7,1-7,25 ppm,<u>dd</u>+<u>d</u>,1+1H; 7,3 ppm,<u>t</u>,1H; 7,40 ppm,<u>d</u>,1H; 7,50 ppm,1H échangeable; 7,65 ppm,<u>d</u>,1H; 7,85 ppm,<u>d</u>,1H; 11 ppm, 1H échangeable |
| 8 | 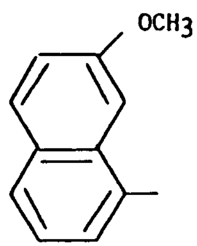 | 2 | -NH-CO 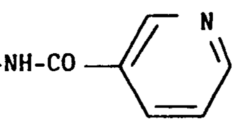 | ($DMSO-d_6$) <u>s</u> RMN-$^1H$ 3,1-4,0 ppm,<u>m</u>+<u>m</u>,2+10H; 7,1-7,25 ppm, <u>m+m</u>,1+ 1H; 7,3 ppm,<u>t</u>,1H; 7,4 ppm,<u>d</u>,1H; 7,6 ppm, <u>d</u>,1H; 7,8 ppm,<u>m</u>,1H; 7,82 ppm, <u>d</u>,1H; 8,65 ppm,<u>d</u>,1H; 8,9 ppm,<u>dd</u>,1H; 9,3 ppm,<u>d</u>,1H; 9,5 ppm,1H échangeable; 11,0-11,3 ppm, 1H échangeable |

TABLEAU I
SUITE 4

| EX. N° | NAPH | n | R1 | Spectre RMN (Solvant) s = sel b = base |
|---|---|---|---|---|
| 9 | 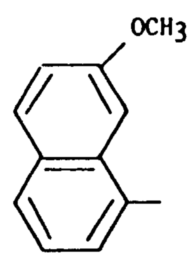 | 4 | 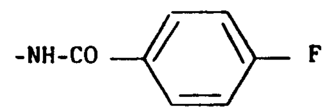 | (DMSO-d6) s RMN-$^1H$ 1,5-1,7 ppm,m,2H; 1,7-2,0 ppm,m,2H; 3,1-3,5 ppm,m,10H; 3,6 ppm,m,2H; 3,65 ppm,1H échangeable; 3,9 ppm,s,3H; 7,05-7,40 ppm, d+t+t+dd+d,1H+2H+1H+1H+1H; 7,6 ppm,d, 1H; 7,85 ppm,d,1H; 7,95 ppm,dd,2H; 10,6-11,0 ppm,1H échangeable |
| 10 | 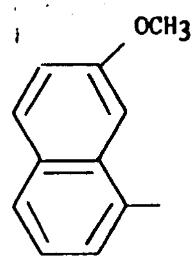 | 2 | 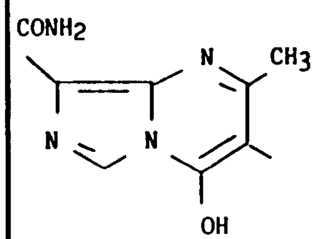 | (DMSO-d6) s RMN-$^1H$ 2,6 ppm,s,3H; 2,9-3,6 ppm,m,10H; 3,75 ppm,m,2H; 3,9 ppm,s,3H; 7,1-7,25 ppm,m, 2H; 7,25 ppm,1H échangeable; 7,3 ppm,t, 1H; 7,40 ppm,d,1H; 7,45 ppm,1H échangeable; 7,6 ppm,d,1H; 7,85 ppm,d,1H; 8,15 ppm,s,1H; 10,8 ppm,1H échangeable, 11,65 ppm,1H échangeable |

TABLEAU I

SUITE 5

| EX. N° | NAPH | n | R1 | Spectre RMN (Solvant) s = sel b = base |
|---|---|---|---|---|
| 11 | 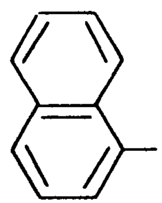 | 2 | 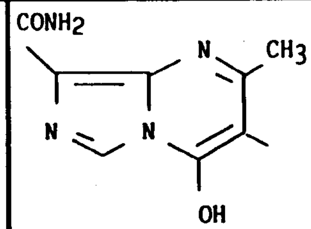 | (DMSO-d6) s RMN-13C 17,3 ppm; 19,5 ppm; 49,4 ppm; 51,3 ppm; 53,9 ppm; 100,1 ppm; 115,0 ppm; 115,1 ppm; 123,1 ppm; 123,5 ppm; 123,9 ppm; 125,7 ppm; 125,8 ppm; 126,0 ppm; 127,8 ppm; 128,3 ppm; 132,5 ppm; 134,2 ppm; 147,6 ppm; 151,4 ppm; 155,8 ppm; 164,3 ppm |
| 12 | 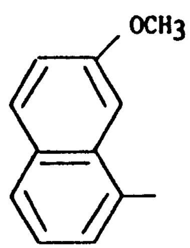 | 2 | 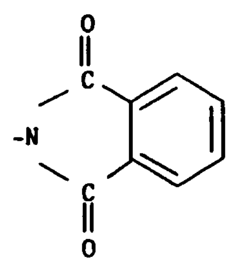 | (DMSO-d6) s RMN-1H 3,1-3,3 ppm,s,2H; 3,3-3,65 ppm,m,6H; 3,75 ppm,m,2H; 3,9 ppm,s,3H; 4,1 ppm,t,2H; 7,15 ppm,d,1H; 7,20 ppm,dd,1H; 7,30 ppm,t,1H; 7,4 ppm,d,1H; 7,6 ppm,d,1H; 7,8-8,0 ppm,m+d,4H+1H; 10,9-11,2 ppm, massif échangeable |

TABLEAU I
SUITE 6

| EX. N° | NAPH | n | $R_1$ | Spectre RMN (Solvant) <u>s</u> = sel <u>b</u> = base |
|---|---|---|---|---|
| 13 | 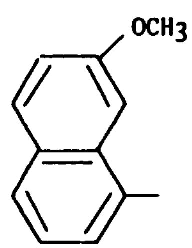 | 2 | - NHCO— 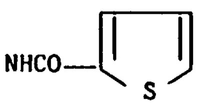 | (DMSO-$d_6$) <u>s</u> RMN-$^1H$ 3,1-3,9 ppm,<u>m</u>,12H; 3,9 ppm,<u>s</u>,3H; 7,1-7,5 ppm,<u>dd</u>+<u>d</u>+<u>m</u>,1H+1H+1H; 7,30 ppm,<u>t</u>,1H; 7,4 ppm,<u>d</u>,1H; 7,60 ppm,<u>d</u>,1H; 7,80 ppm,<u>d</u>,1H; 7,85 ppm,<u>d</u>,1H; 7,95 ppm,<u>d</u>,1H; 9,1 ppm, massif échangeable; 11 ppm, massif échangeable |

**ETUDE PHARMACOLOGIOUE**

**EXEMPLE 20**

**Evaluation de l'activité antihypertensive des composés de l'invention**

Des chiens batards (mâles et femelles) sont anesthésiés au phénobarbital (30 mg/kg i.v.) puis placés en respiration artificielle (respirateur Bird Mark VII). La pression artérielle est mesurée au moyen d'un cathéther placé dans l'aorte abdominale via l'artère fémorale. Ce cathéter est connecté à une cellule de pression (Statham ® $R_{23}D_6$) relié à un enregistreur.

La fréquence cardiaque est mesurée au moyen d'un Gould Biotach ®.

L'activité nerveuse sympathique est enregistrée au niveau du nerf rénal au moyen d'électrode d'argent. Le signal amplifié est visualisé sur un oscilloscope (Tektronix 5115 ®) puis mesuré en µV au moyen d'un intégrateur Gould. Les composés à examiner sont administrés par voie i.v. Les résultats de l'étude sont donnés dans le Tableau II.

**TABLEAU II**

| COMPOSES | DOSE µg/kg | EFFET SUR LA PRESSION ARTERIELLE (mm/Hg) | EFFET SUR LE RYTHME CARDIAQUE (Batt/min.) |
|---|---|---|---|
| Flesinoxan Isomère (+) | 10<br>30<br>100 | ↘ # 10<br>↘ 10-25<br>↘ > 30 | ↘ # 15<br>↘ 15-25<br>↘ > 40 |
| Flesinoxan racémique | 10<br>30<br>100<br>300 | 0<br>↘ # 10<br>↘ 10-25<br>↘ > 30 | 0<br>0<br>↘ # 15<br>↘ 15-25 |
| EXEMPLE 3 | 3<br>10<br>30 | ↘ # 10<br>↘ 10-25<br>↘ > 30 | ↘ # 15<br>↘ 15-25<br>↘ 30-40 |
| EXEMPLE 5 | 3<br>10<br>30 | ↘ # 10<br>↘ 10-25<br>↘ 10-25 | ↘ # 15<br>↘ 30-40<br>↘ 30-40 |
| EXEMPLE 13 | 1<br>3<br>10 | ↘ # 10<br>↘ 10-25<br>↘ > 30 | ↘ 15-25<br>↘ 15-25<br>↘ 15-25 |

Les résultats indiqués dans le Tableau II démontrent que les composés de l'invention sont environ 3-10 fois plus puissants que le produit de référence flesinoxan sous forme racémique ou sous forme d'isomère (+). Cet isomère est l'isomère le plus actif du flesinoxan.

Concernant l'activité nerveuse sympathique, les résultats obtenus avec le composé de l'exemple 3 après administration d'une dose de 10 µg/kg par voie i.v. sont donnés dans la Figure 1.

**EXEMPLE 21**

**Evaluation de l'affinité pour les récepteurs 5-$HT_{1A}$**

Pour les essais, on a utilisé du tissu de l'hippocampe obtenu à partir de rats-Wistar décapités. Les animaux ont été sacrifiés 48 heures avant l'expérience et les tissus isolés ont été conservés à -86 °C. Pour

la préparation des membranes, les tissus ont été ensuite homogénisés en utilisant 20 volumes d'une solution tampon 50 mM Tris HCl (pH = 7,7 ajusté à l'aide de $NH_4Cl$ à 25 ° C) pour un volume de tissus, à une température voisine de 0 ° C, avec un homogénisateur Polytron ®, puis, le tout a été centrifugé. (35 000 g x 20 min à 4 ° C). Le culot ainsi obtenu a été suspendu dans 100 volumes d'une solution tampon d'incubation. (Tris 60 mM, pargyline 10 µM, $CaCl_2$ 4mM et acide ascorbique 0,1% (p/v); pH ajusté à 7,7 avec HCl 5N). Les composés à examiner ont été aussi dilués dans le tampon d'incubation puis les solutions essais ont été préparées en ajoutant dans des tubes de verre de 12 x 75 mm, 100 µl d'une solution du composé à examiner, 100 µl d'une solution de [3H] 8-OH-DPAT C = 0,4 nM (radioactivité spécifique = 205 Ci/mmol). Le binding non spécifique a été déterminé à l'aide d'une solution de 5-hydroxytryptamine 10 µm et correspond à 5-10% du binding total.

Les tubes ont été incubés pendant 30 min à 37 ° C, et les solutions ont été ensuite filtrées sur des filtres en fibres de verre GF/B traités avec 0,1% de polyéthylèneimine (Whatman ®). Les filtres ont été rincés 2 fois avec 5 ml de la solution tampon d'incubation puis, ont été placés dans des ampoules dans lesquelles ont été ajoutés 4,5 ml de "Picofluor scintillation fluid" ® (Packard). La radioactivité a été déterminée en utilisant des standards externes.

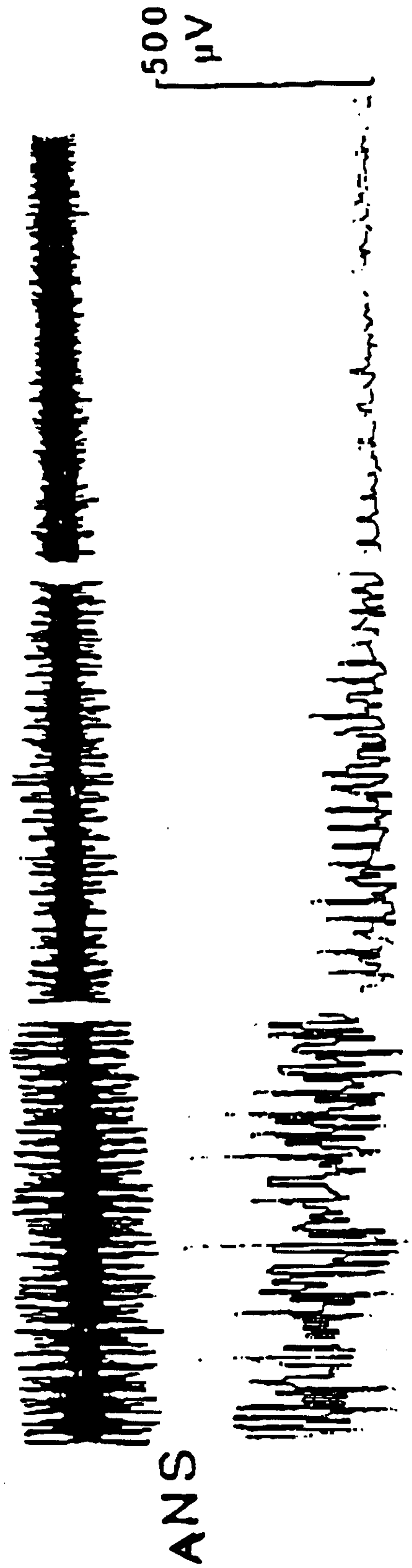


FIGURE 1
Composé de l'exemple 18
30 µg/kg i.v.

Les pKi ont été évalués en utilisant l'équation de Cheung-Prusoff :

$$-\log (IC_{50} / [1 + [3H]\ 8\text{-}OH - DPAT] / Kd).$$

Les composés de l'invention ont une grande affinité pour les sites 5-$HT_{1A}$. Les pKi des composés de l'invention sont de l'ordre de 9.01 nMoles/ litre.

**PREPARATION PHARMACEUTIOUE**

**EXEMPLE 22**

**Gélules dosées à 1 mg de chlorhydrate de la 1-(7-méthoxynapt-a-yl) [4-[2-(4-fluorobenzoyl) aminoéthyl] pipérazine [M.N.F.B.A.E.P.]**

| | |
|---|---|
| M.N.F.B.A.E.P. | 1 mg |
| Amidon de maïs | 15 mg |
| Lactose | 25 mg |
| Talc | 5 mg |

**Revendications**

**Revendications pour les Etats contractants suivants : AT, BE, CH, DE, DK, FR, GB, IT, LI, LU, NL, SE**

**1.** Composés de formule I :

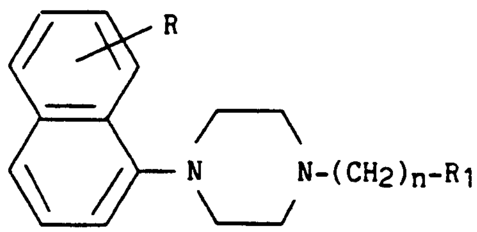

(I)

dans laquelle :

- n représente un nombre entier de 1 à 4,
- R représente un atome d'hydrogène, un atome d'halogène, un radical alkyle de 1 à 6 atomes de carbone, un radical hydroxy, ou un radical alcoxy de 1 à 6 atomes de carbone,
- $R_1$ représente un radical de formule $A_1$ :

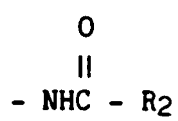

($A_1$)

(dans laquelle

- $R_2$ représente un radical alkyle de 1 à 6 atomes de carbone, un radical cycloalkyle de 3 à 7 atomes de carbone, un radical phényle -éventuellement substitué par un ou plusieurs atomes d'halogène, par un ou plusieurs radicaux alkyle de 1 à 6 atomes de carbone ou par un ou plusieurs radicaux alcoxy de 1 à 6 atomes de carbone-, un radical -O-alkyle de 1 à 6 atomes de carbone, un radical 3-pyridyle, un radical 2-pyrrolyle, un radical quinolyle, un radical 1-isoquinolyle, un radical 2-thiényle, un radical 3-indolyle),

un radical de formule $A_2$ :

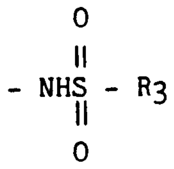

(A2)

(dans laquelle $R_3$ représente un radical alkyle de 1 à 6 atomes de carbone, un radical cycloalkyle de 3 à 7 atomes de carbone ou un radical phényle -éventuellement substitué par un ou plusieurs atomes d'halogène, par un ou plusieurs radicaux alkyle de 1 à 6 atomes de carbone ou par un ou plusieurs radicaux alcoxy de 1 à 6 atomes de carbone-),

un radical de formule $A_3$ :

$-NHCONHR_4$ **(A3)**

(dans laquelle $R_4$ représente un radical alkyle de 1 à 6 atomes de carbone ou un radical phényle éventuellement substitué par un ou plusieurs atomes d'halogène, par un ou plusieurs radicaux alkyle ou alcoxy de 1 à 6 atomes de carbone),

un radical o-sulfobenzoïque imido de formule $A_5$ :

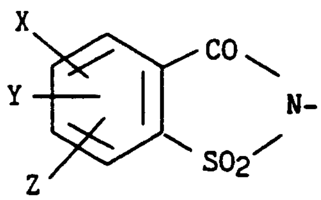

(A5)

ou un radical de formule $A_5$ :

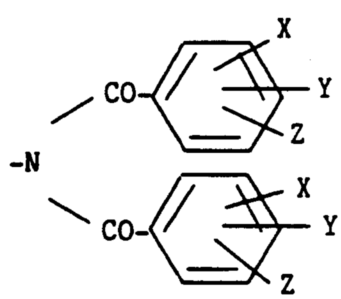

(A6)

(dans lesquelles X, Y et Z, identiques ou différents, représentent chacun un atome d'hydrogène, un atome d'halogène, un radical alkyle de 1 à 6 atomes de carbone ou un radical alcoxy de 1 à 6 atomes de carbone),

ou un radical de formule $A_7$ :

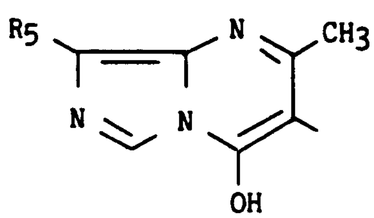

(A7)

(dans laquelle $R_5$ représente un radical carbamoyle, un radical cyano, un radical carboxy, ou un

radical alcoxycarbonyle de 1 à 6 atomes de carbone),
ou un radical de formule $A_8$ :

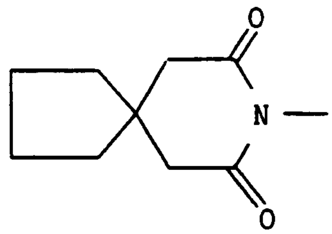

**(A8)**

leurs stéréoisomères et leurs sels d'addition à un acide minéral ou organique pharmaceutiquement acceptable.

**2.** La 1-(7-méthoxynapht-1-yl) 4-[2-(4-fluorobenzoylamino)éthyl] pipérazine, composé répondant à la formule I selon la revendication 1 et ses sels d'addition à un acide minéral ou organique, pharmaceutiquement acceptable.

**3.** La 1-(7-hydroxynapht-1yl) 4-[2-(4-fluorobenzoylamino)éthyl] pipérazine, composé répondant à la formule I selon la revendication 1 et ses sels d'addition à un acide minéral ou organique, pharmaceutiquement acceptable.

**4.** La 1-(7-méthoxynapht-1-yl) 4-[2-(thien-2-oyl amino) éthyl] pipérazine, composé répondant à la formule I selon la revendication 1 et ses sels d'addition à un acide minéral ou organique, pharmaceutiquement acceptable.

**5.** La 1-(7-méthoxynapht-1-yl)-4-(2-butyrylaminoéthyl) pipérazine, composé répondant à la formule I selon la revendication 1 et ses sels d'addition à un acide minéral ou organique, pharmaceutiquement acceptable.

**6.** Procédé de préparation des composés de la formule générale I, selon la revendication 1, caractérisé en ce que l'on utilise comme matière première :
**a/ <u>ou bien</u>**
composé de formule II :

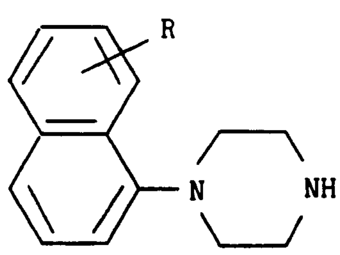

**(II)**

dans laquelle R a la même signification que pour la formule I, selon la revendication 1,
que l'on condense

**<u>soit</u>**

avec un nitrile de formule III :

$Hal(CH_2)_{n-1}CN$ **(III)**

dans laquelle Hal représente un atome d'halogène et n a la même signification que pour la formule

I, selon la revendication 1, dans un solvant organique, à température ambiante, en présence d'un sel d'un métal alcalin pour obtenir les composés de formule IV :

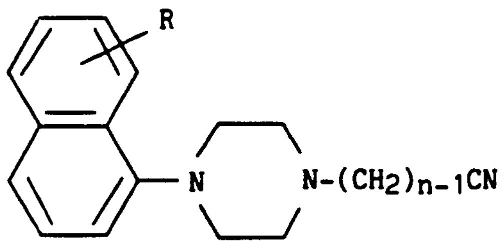

(IV)

dans laquelle R et n ont la même signification que pour la formule I, selon la revendication 1, que l'on transforme à l'aide de l'hydrure de lithium et d'aluminium ou d'un autre réactif chimique équivalent, à un composé de formule V :

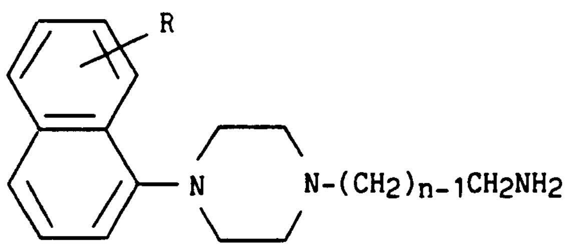

(V)

dans laquelle R et n ont la même signification que pour la formule I, selon la revendication 1, que l'on fait réagir

ou

avec une quantité équimolaire d'un composé de formule $VI_A$ ou $VI_B$ :

$ClCOR_2$ **($VI_A$)**

$ClSO_2R_3$ **($VI_B$)**

dans lesquelles $R_2$ et $R_3$ ont les mêmes significations que pour la formule I, selon la revendication 1, pour obtenir respectivement les composés de formule I dans laquelle $R_1$ représente un radical de formule $A_1$, et les composés de formule I dans laquelle $R_1$ représente un radical de formule $A_2$,

composés de formule I dans laquelle $R_2$ représente un radical -O-alkyle, que l'on fait réagir, si on le souhaite, avec un composé de formule VII :

$R_4NH_2$ **(VII)**

dans laquelle $R_4$ a la même signification que pour la formule I, selon la revendication 1, pour former les composés de formule I dans laquelle $R_1$ représente un radical de formule $A_3$,

ou

avec un composé de formule VIII, en excès, :

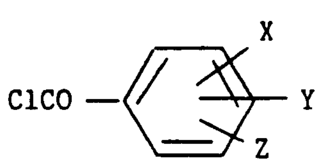

(VIII)

dans laquelle X, Y et Z ont la même signification que pour la formule I, selon la revendication 1, pour former un composé de formule 1, selon la revendication I, dans laquelle $R_1$ représente un

radical de formule $A_6$,

**soit**

avec un composé de formule $IX_B$ :

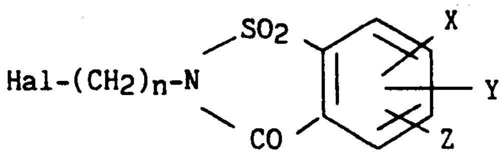

**($IX_B$)**

dans laquelle Hal a la même signification que pour la formule III et X, Y et Z ont la même signification que pour la formule I, selon la revendication 1,

pour former les composés de formule I, selon la revendication 1, dans laquelle $R_1$ est un radical de formule $A_6$,

**soit**

avec un alcool de formule X :

$Hal(CH_2)_nOH$ **(X)**

dans laquelle n a la même signification que pour la formule I, selon la revendication 1, et la signification de Hal reste identique à celle donnée pour la formule III,

pour former les composés de formule XI :

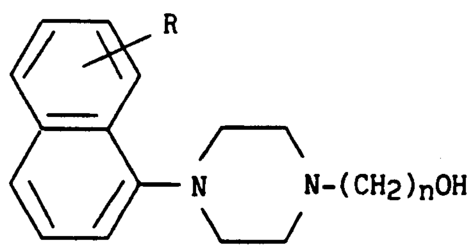

**(XI)**

dans laquelle R et n ont la même signification que pour la formule I, selon la revendication 1,

que l'on soumet à l'action du chlorure de thionyle ou d'un autre réactif chimique équivalent pour former les composés de formule XII :

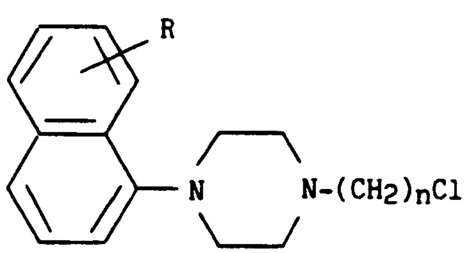

**(XII)**

dans laquelle R et n ont la même signification que pour la formule I, selon la revendication 1,

que l'on condense

ou

avec l'acétoacétate d'éthyle pour former un composé de formule XIII,

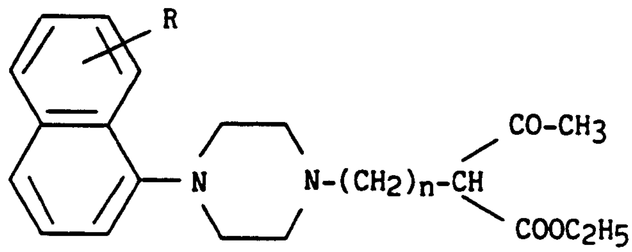

(XIII)

dans laquelle n et R ont la même signification que pour la formule I, selon la revendication 1, que l'on condense avec un dérivé de 4-amino imidazole de formule XIV :

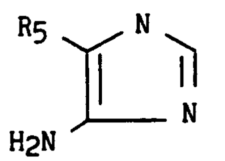

(XIV)

dans laquelle $R_5$ a la même signification que pour la formule I, selon la revendication 1,

pour former les composés de formule I, selon la revendication 1, dans laquelle $R_1$ représente un radical de formule $A_7$,

ou

avec un composé de formule XV

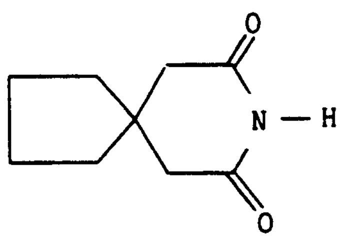

(XV)

pour former les composés de formule I dans laquelle $R_1$ représente un radical de formule $A_8$,

**b/ ou bien** :

un composé de formule XVI :

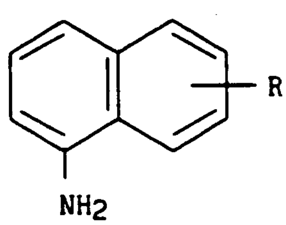

(XVI)

dans laquelle R a la même signification que dans la formule I,

que l'on condense avec de l'acide N-benzyliminodiacétique de formule XVII préalablement mis à reflux avec du carbonyldiimidazole dans un solvant anhydre comme le tétrahydrofurane

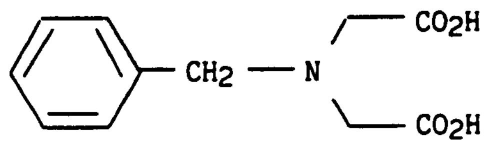

(XVII)

pour conduire à une pipérazine-2,6-dione de formule XVIII :

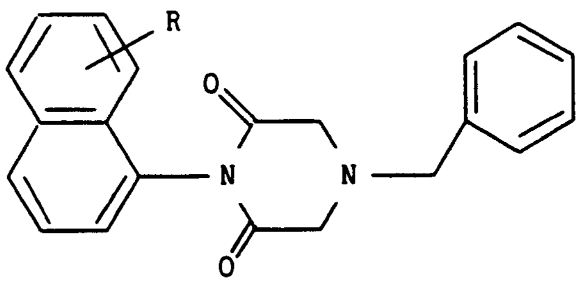

(XVIII)

dans laquelle R a la même signification que dans la formule I,

sur laquelle on réalise une hydrogénation catalytique en présence de charbon palladié comme catalyseur,

pour conduire à une pipérazine-2,6-dione de formule XIX :

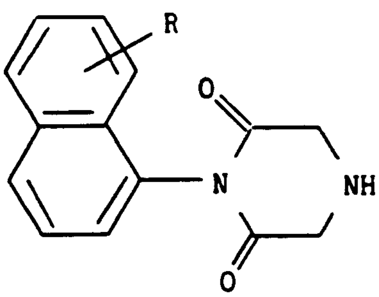

(XIX)

dans laquelle R a la même signification que dans la formule I,

que l'on condense

<u>soit</u>

avec un nitrile de formule III :

**Hal - $(CH_2)_{n-1}$ - CN (III)**

dans laquelle Hal et n ont la même signification que précédemment,

pour conduire à une pipérazine-2,6-dione de formule XX :

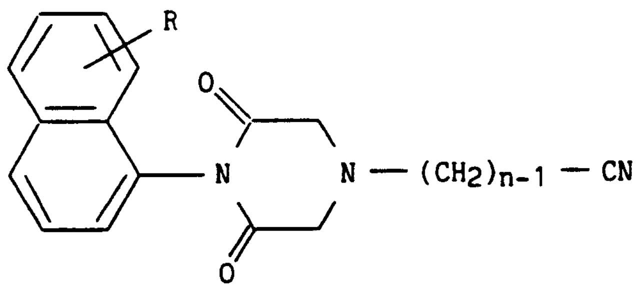

(XX)

dans laquelle R et n ont la même signification que dans la formule I, que l'on réduit en présence du complexe borane-diméthylsulfure pour conduire à une pipérazine de formule V :

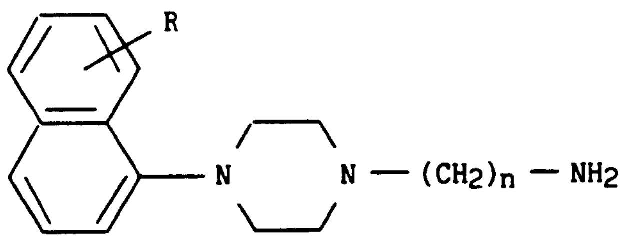

(V)

dans laquelle R et n ont la même signification que dans la formule I, que l'on fait réagir

ou

avec une quantité équimoléculaire d'un composé de formule $VI_A$ ou $VI_B$ :

**Cl CO $R_2$ ($VI_A$)**

**Cl $SO_2$ $R_3$ ($VI_B$)**

dans lesquelles $R_2$ et $R_3$ ont les mêmes significations que pour la formule I, pour obtenir respectivement les composés de formule I dans laquelle $R_1$ représente un radical de formule $A_1$, et les composés de formule I dans laquelle $R_1$ représente un radical de formule $A_2$,

composés de formule I dans laquelle $R_2$ représente un radical - O-alkyle, que l'on fait réagir, si on le souhaite, avec un composé de formule VII :

$R_4NH_2$ **(VII)**

dans laquelle $R_4$ a la même signification que pour la formule I, pour former les composés de formule I dans laquelle $R_1$ représente un radical de formule $A_3$,

ou

avec un composé de formule VIII, en excès, :

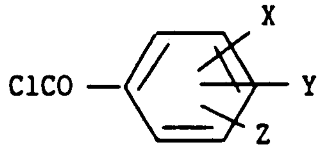

(VIII)

dans laquelle X, Y et Z ont la même signification que pour la formule I, pour former un composé de formule I dans laquelle $R_1$ représente un radical de formule $A_6$,

soit

avec un alcool de formule X :

**Hal $(CH_2)_n$ OH (X)**

dans laquelle Hal et n ont la même signification que précédemment,
pour conduire à une pipérazine-2,6-dione de formule XXI :

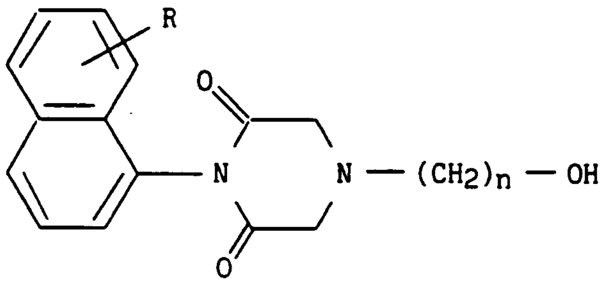

(XXI)

dans laquelle R et n ont la même signification que dans la formule I,
que l'on soumet à l'action de chlorure de thionyle ou d'un autre réactif chimique équivalent,
pour conduire à une pipérazine-2,6-dione de formule XXII :

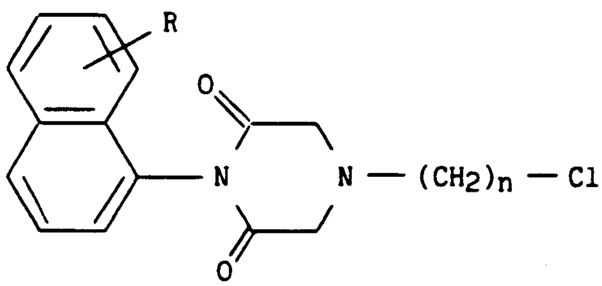

(XXII)

dans laquelle R et n ont la même signification que dans la formule I
que l'on réduit en présence du complexe borane-diméthylsulfure
pour conduire à une pipérazine de formule XII :

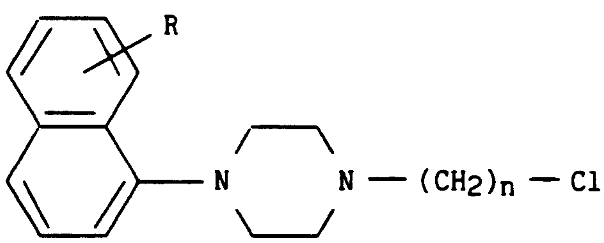

(XII)

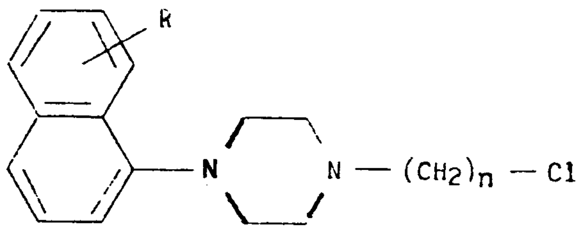

(XII)

ou

avec un composé de formule $XXIII_B$ ou XV

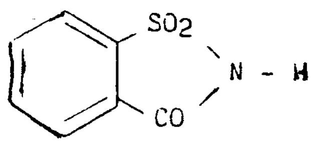

$(XXIII_B)$

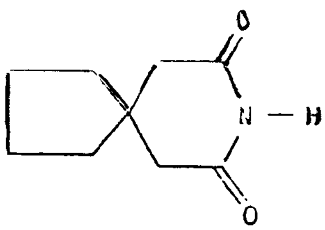

(XV)

pour conduire respectivement aux composés de formule I dans laquelle $R_1$ est radical, $A_5$ ou $A_5$,

ou

avec l'acétoacétate d'éthyle pour forme un composé de formule XIII

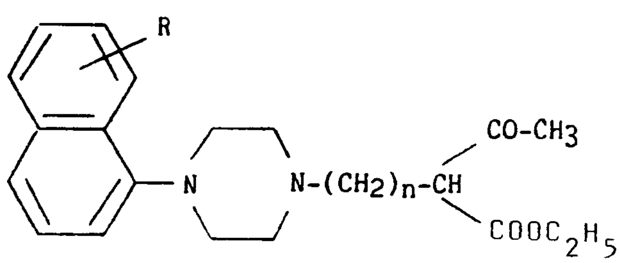

(XIII)

dans laquelle R et n ont la même signification que dans la formule I, que l'on condense

ou

avec un composé de formule, $XXIII_B$ ou XV

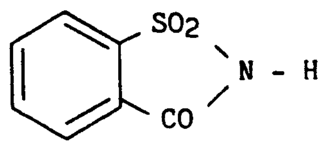

(XXIII$_B$)

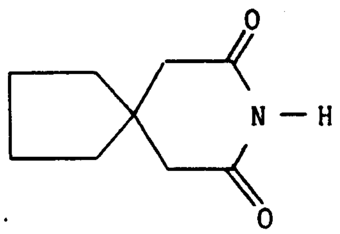

(XV)

pour conduire respectivement aux composés de formule I dans laquelle $R_1$ est un radical $A_5$ ou $A_5$,

ou

avec l'acétoacétate d'éthyle pour former un composé de formule XIII

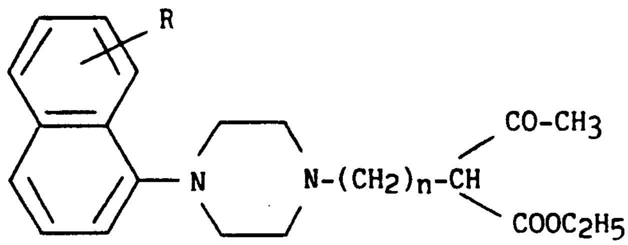

(XIII)

dans laquelle n et R ont la même signification que pour la formule I,
que l'on condense avec un dérivé de 4-amino imidazole de formule XIV :

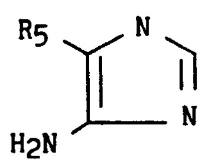

(XIV)

dans laquelle $R_5$ a la même signification que pour la formule I,
pour former les composés de formule I dans laquelle $R_1$ représente un radical de formule $A_7$,

**lesquels, composés de formule I, ensuite,**

si l'on désire, sont salifiés par un acide organique ou minéral, pharmaceutiquement acceptable, pour former les sels d'addition correspondants.

**7.** Composition pharmaceutique contenant comme principe actif un composé selon l'une quelconque des revendications 1 à 4, en association ou en mélange avec un excipient ou un véhicule inerte non toxique, pharmaceutiquement acceptable.

**8.** Composition pharmaceutique selon la revendication 6 renfermant le principe actif à la dose de 0,05 à 10 mg.

**9.** Composition pharmaceutique selon les revendications 6 et 7 renfermant comme principe actif au moins un composé selon les revendications 1 à 4 utilisable dans le traitement des maladies nécessitant des

agonistes ou des antagonistes des récepteurs 5-$HT_{1A}$.

**10.** Composition pharmaceutique selon les revendications 6 et 7 renfermant comme principe actif au moins un composé selon les revendications 1 à 4 utilisable comme antihypertenseur, anxiolytique et antidépresseur.

**Revendications pour les Etats contractants suivants : ES, GR**

**1.** Procédé de préparation des composés de formule I :

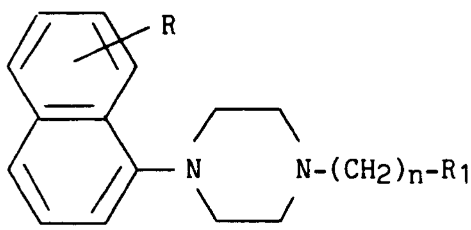

**(I)**

dans laquelle :

- n représente un nombre entier de 1 à 4,
- R représente un atome d'hydrogène, un atome d'halogène, un radical alkyle de 1 à 6 atomes de carbone, un radical hydroxy, ou un radical alcoxy de 1 à 6 atomes de carbone,
- $R_1$ représente un radical de formule $A_1$ :

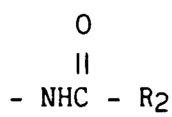

**($A_1$)**

(dans laquelle

- $R_2$ représente un radical alkyle de 1 à 6 atomes de carbone, un radical cycloalkyle de 3 à 7 atomes de carbone, un radical phényle -éventuellement substitué par un ou plusieurs atomes d'halogène, par un ou plusieurs radicaux alkyle de 1 à 6 atomes de carbone ou par un ou plusieurs radicaux alcoxy de 1 à 6 atomes de carbone-, un radical -O-alkyle de 1 à 6 atomes de carbone, un radical 3-pyridyle, un radical 2-pyrrolyle, un radical quinolyle, un radical 1-isoquinolyle, un radical 2-thiényle, un radical 3-indolyle),

un radical de formule $A_2$ :

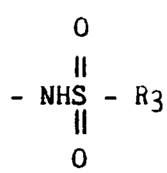

**($A_2$)**

(dans laquelle $R_3$ représente un radical alkyle de 1 à 6 atomes de carbone, un radical cycloalkyle dc 3 à 7 atomes de carbone ou un radical phényle -éventuellement substitué par un ou plusieurs atomes d'halogène, par un ou plusieurs radicaux alkyle de 1 à 6 atomes de carbone ou par un ou plusieurs radicaux alcoxy de 1 à 6 atomes de carbone-),

un radical de formule $A_3$ :

- $NHCONHR_4$ **(A3)**

(dans laquelle $R_4$ représente un radical alkyle de 1 à 6 atomes de carbone ou un radical phényle éventuellement substitué par un ou plusieurs atomes d'halogène, par un ou plusieurs radicaux alhyle ou

alcoxy de 1 à 6 atomes de carbone),
un radical o-sulfobenzoïque imido de formule $A_5$ :

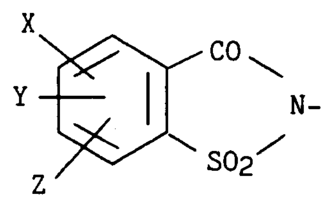

(A5)

ou un radical de formule $A_5$ :

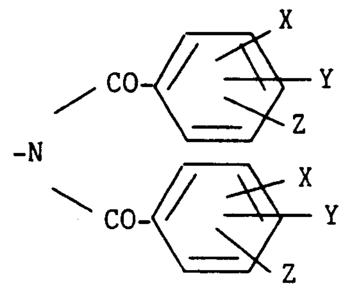

(A6)

(dans lesquelles X, Y et Z, identiques ou différents, représentent chacun un atome d'hydrogène, un atome d'halogène, un radical alkyle de 1 à 6 atomes de carbone ou un radical alcoxy de 1 à 6 atomes de carbone),
ou un radical de formule $A_7$ :

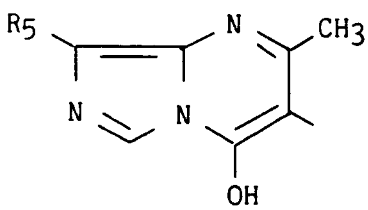

(A7)

(dans laquelle $R_5$ représente un radical carbamoyle, un radical cyano, un radical carboxy, ou un radical alcoxycarbonyle de 1 à 6 atomes de carbone),
ou un radical de formule $A_5$ :

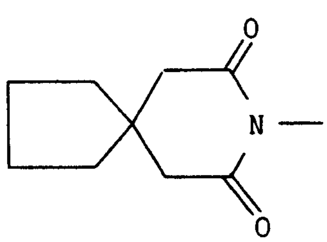

(A8)

leurs stéréoisomères et leurs sels d'addition à un acide minéral ou organique pharmaceutiquement acceptable,
caractérisé en ce que l'on utilise comme matière première :
**a/ ou bien**
composé de formule II :

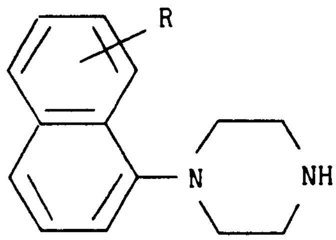

(II)

dans laquelle R a la même signification que pour la formule I, selon la revendication 1, que l'on condense

soit

avec un nitrile de formule III :

$Hal(CH_2)_{n-1}CN$ **(III)**

dans laquelle Hal représente un atome d'halogène et n a la même signification que pour la formule I, selon la revendication 1, dans un solvant organique, à température ambiante, en présence d'un sel d'un métal alcalin pour obtenir les composés de formule IV :

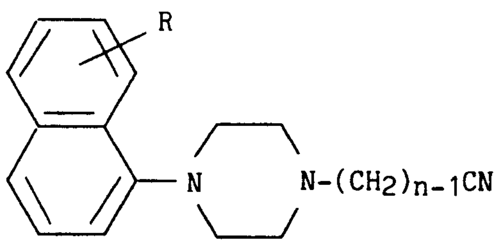

(IV)

dans laquelle R et n ont la même signification que pour la formule I, selon la revendication 1, que l'on transforme à l'aide de l'hydrure de lithium et d'aluminium ou d'un autre réactif chimique équivalent, à un composé de formule V :

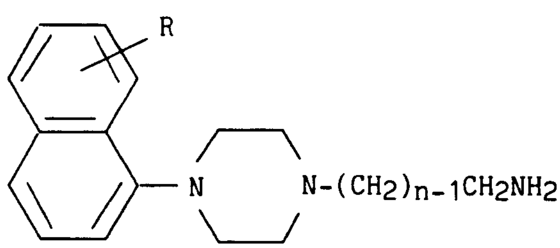

(V)

dans laquelle R et n ont la même signification que pour la formule I, selon la revendication 1, que l'on fait réagir

ou

avec une quantité équimolaire d'un composé de formule $VI_A$ ou $VI_B$ :

$ClCOR_2$ **($VI_A$)**

$ClSO_2R_3$ **($VI_B$)**

dans lesquelles $R_2$ et $R_3$ ont les mêmes significations que pour la formule I, selon la revendication 1, pour obtenir respectivement les composés de formule I dans laquelle $R_1$ représente un radical de formule $A_1$, et les composés de formule I dans laquelle $R_1$ représente un radical de formule $A_2$,

composés de formule I dans laquelle $R_2$ représente un radical -O-alkyle, que l'on fait réagir, si on le souhaite, avec un composé de formule VII :

$R_4NH_2$ **(VII)**

dans laquelle $R_4$ a la même signification que pour la formule I, selon la revendication 1,

pour former les composés de formule I dans laquellc $R_1$ représente un radical de formule $A_3$,

<u>ou</u>

avec un composé de formule VIII, en excès, :

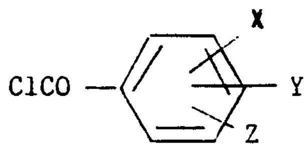

**(VIII)**

dans laquelle X, Y et Z ont la même signification que pour la formule I, selon la revendication 1,

pour former un composé de formule I, selon la revendication 1, dans laquelle $R_1$ représente un radical de formule $A_6$,

**<u>soit</u>**

avec un composé de formule $IX_B$ :

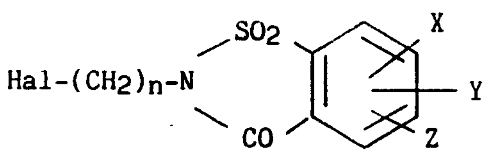

**($IX_B$)**

dans laquelle Hal a la même signification que pour la formule III et X, Y et Z ont la même signification que pour la formule I, selon la revendication 1,

pour former respectivement les composés de formule 1, selon la revendication 1, dans laquelle $R_1$ est un radical de formule $A_6$,

**<u>soit</u>**

avec un alcool de formule X :

$Hal(CH_2)_nOH$ **(X)**

dans laquelle n a la même signification que pour la formule I, selon la revendication 1, et la signification de Hal reste identique à celle donnée pour la formule III,

pour former les composés de formule XI :

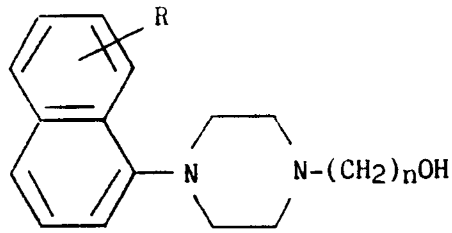

(XI)

dans laquelle R et n ont la même signification que pour la formule I, selon la revendication 1,

que l'on soumet à l'action du chlorure de thionyle ou d'un autre reactif chimique équivalent pour former les composés de formule XII :

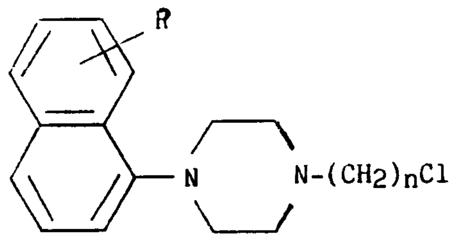

(XII)

dans laquelle R et n ont la même signification que pour la formule I, selon la revendication 1,

que l'on condense

ou

avec l'acétoacétate d'éthyle pour former un composé de formule XIII,

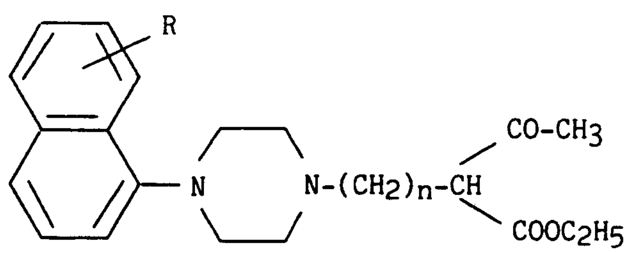

(XIII)

dans laquelle n et R ont la même signification que pour la formule I, selon la revendication 1,

que l'on condense avec un dérivé de 4-amino imidazole de formule XIV :

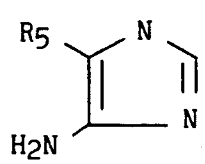

(XIV)

dans laquelle $R_5$ a la même signification que pour la formule I, selon la revendication 1,

pour former les composés de formule I, selon la revendication 1, dans laquelle $R_1$ représente un radical de formule $A_7$,

ou

avec un composé de formule XV

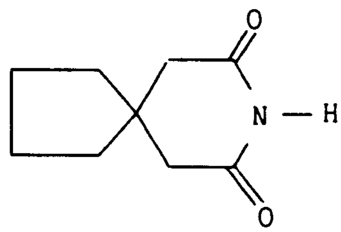

(XV)

pour former les composés de formule I dans laquelle $R_1$ représente un radical de formule $A_8$,

**b/ ou bien** :

un composé de formule XVI :

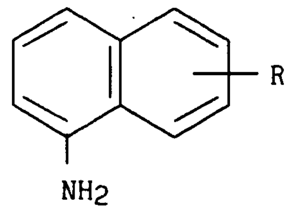

(XVI)

dans laquelle R a la même signification que dans la formule I,

que l'on condense avec de l'acide N-benzyliminodiacétique de formule XVII préalablement mis à reflux avec du carbonyldiimidazole dans un solvant anhydre comme le tétrahydrofurane

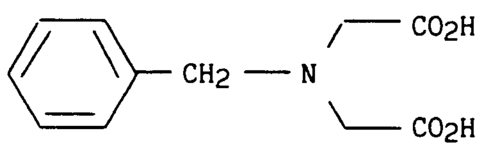

(XVII)

pour conduire à une pipérazine-2,6-dione de formule XVIII :

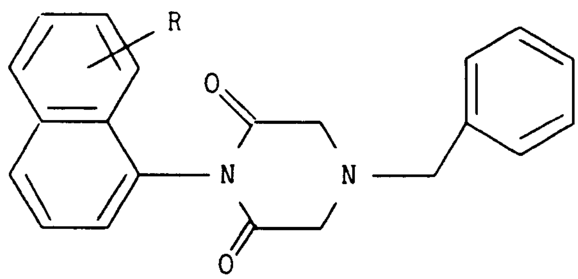

(XVIII)

dans laquelle R a la même signification que dans la formule I,

sur laquelle on réalise une hydrogénation catalytique en présence de charbon palladié comme catalyseur,

pour conduire à une pipérazine-2,6-dione de formule XIX : dans laquelle R a la même signification que dans la formule I,

que l'on condense

soit

avec un nitrile de formule III :

**Hal - $(CH_2)_{n-1}$ - CN (III)**

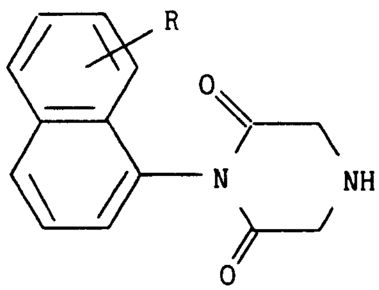

**(XIX)**

dans laquelle Hal et n ont la même signification que précédemment, pour conduire à une pipérazine-2,6-dione de formule XX :

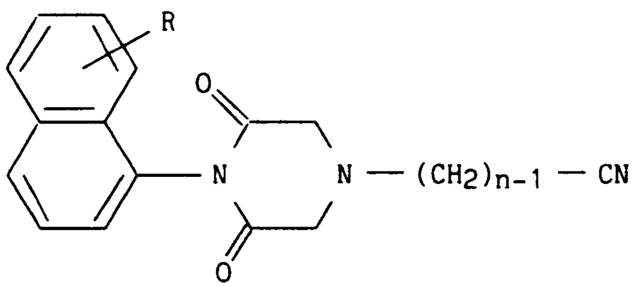

**(XX)**

dans laquelle R et n ont la même signification que dans la formule I, que l'on réduit en présence du complexe borane-diméthylsulfure pour conduire à une pipérazine de formule V :

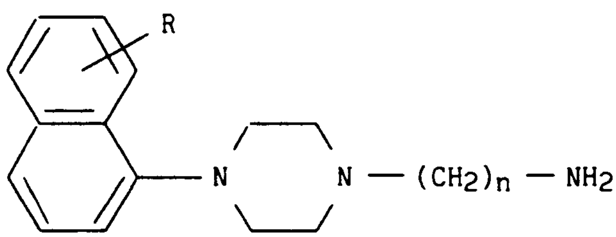

**(V)**

dans laquelle R et n ont la même signification que dans la formule I, que l'on fait réagir

ou

avec une quantité équimoléculaire d'un composé de formule $VI_A$ ou $VI_B$ :

**Cl CO $R_2$ ($VI_A$)**

**Cl $SO_2$ $R_3$ ($VI_B$)**

dans lesquelles $R_2$ et $R_3$ ont les mêmes significations que pour la formule I, pour obtenir respectivement les composés de formule I dans laquelle $R_1$ représente un radical de formule $A_1$, et les composés de formule I dans laquelle $R_1$ représente un radical de formule $A_2$,

composés de formule I dans laquelle $R_2$ représente un radical -O-alkyle, que l'on fait réagir, si on le souhaite, avec un composé de formule VII :

$R_4NH_2$ **(VII)**

dans laquelle $R_4$ a la même signification que pour la formule I,
pour former les composés de formule I dans laquelle $R_1$ représente un radical de formule $A_3$,

ou

avec un composé de formule VIII, en excès, :

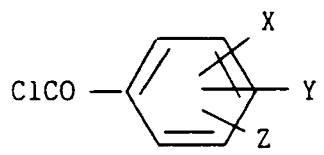

(VIII)

dans laquelle X, Y et Z ont la même signification que pour la formule I,
pour former un composé de formule I dans laquelle $R_1$ représente un radical de formule $A_6$,

soit

avec un alcool de formule X :

**Hal $(CH_2)_n$ OH (X)**

dans laquelle Hal et n ont la même signification que précédemment,
pour conduire à une pipérazine-2,6-dione de formule XXI :

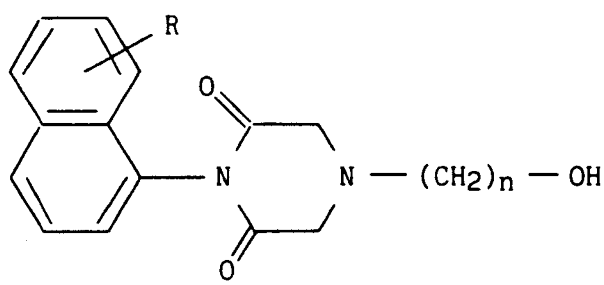

(XXI)

dans laquelle R et n ont la même signification que dans la formule I,
que l'on soumet à l'action de chlorure de thionyle ou d'un autre réactif chimique équivalent,
pour conduire à une pipérazine-2,6-dione de formule XXII :

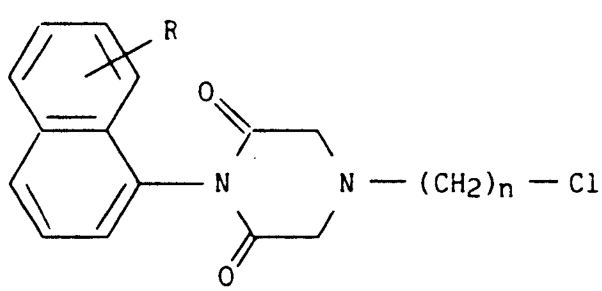

(XXII)

dans laquelle R et n ont la même signification que dans la formule I
que l'on réduit en présence du complexe borane-diméthylsulfure
pour conduire à une pipérazine de formule XII :
dans laquelle R et n ont la même signification que dans la formule I,
que l'on condense
dans laquelle n et R ont la même signification que pour la formule I,
que l'on condense avec un dérivé de 4-amino imidazole de formule XIV :

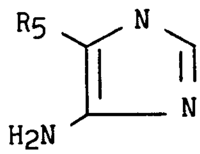

(XIV)

dans laquelle $R_5$ a la même signification que pour la formule I,
pour former les composés de formule I dans laquelle $R_1$ représente un radical de formule $A_7$,

lesquels, composés de formule I, ensuite,

si l'on désire, sont salifiés par un acide organique ou minéral, pharmaceutiquement acceptable, pour former les sels d'addition correspondants.

**2.** Procédé de préparation selon la revendication 1 de la 1-(7-méthoxynapht-1-yl) 4-[2-(4-fluorobenzoylamino)éthyl] pipérazine et ses sels d'addition à un acide minéral ou organique, pharmaceutiquement acceptable.

**3.** Procédé de préparation selon la revendication 1 de la 1-(7-hydroxynapht-1-yl) 4-[2-(4-fluorobenzoylamino)éthyl] pipérazine et ses sels d'addition à un acide minéral ou organique, pharmaceutiquement acceptable.

**4.** Procédé de préparation selon la revendication 1 de la 1-(7-méthoxynapht-1-yl) 4-[2-(thien-2-oyl amino) éthyl] pipérazine et ses sels d'addition à un acide minéral ou organique, pharmaceutiquement acceptable.

**5.** Procédé de préparation selon la revendication 1 de la 1-(7-méthoxynapht-1-yl)-4-(2-butyrylaminoéthyl) pipérazine et ses sels d'addition à un acide minéral ou organique, pharmaceutiquement acceptable.

**Claims**

**Claims for the following Contracting states : AT, BE, CH, DE, DK, FR, GB, IT, LI, LU, NL, SE**

**1.** Compounds of formula I:

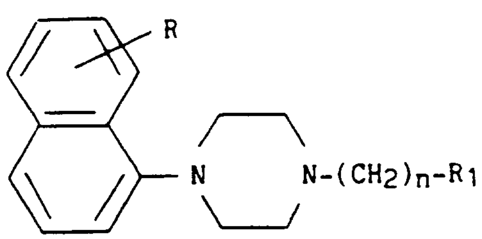

(I)

in which:

- n represents an integer from 1 to 4;
- R represents a hydrogen atom, a halogen atom, an alkyl radical having from 1 to 6 carbon atoms, a hydroxy radical, or an alkoxy radical having from 1 to 6 carbon atoms; and
- $R_1$ represents a radical of the formula $A_1$:

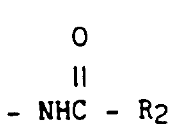

($A_1$)

(in which $R_2$ represents an alkyl radical having from 1 to 6 carbon atoms, a cycloalkyl radical

having from 3 to 7 carbon atoms, a phenyl radical optionally substituted by one or more halogen atoms, by one or more alkyl radicals having from 1 to 6 carbon atoms or by one or more alkoxy radicals having from 1 to 6 carbon atoms, an -O-alkyl radical having from 1 to 6 carbon atoms, a 3-pyridyl radical, a 2-pyrrolyl radical, a quinolyl radical, a 1-isoquinolyl radical, a 2-thienyl radical or a 3-indolyl radical),
a radical of the formula $A_2$:

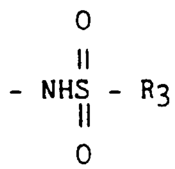

$(A_2)$

(in which $R_3$ represents an alkyl radical having from 1 to 6 carbon atoms, a cycloalkyl radical having from 3 to 7 carbon atoms or a phenyl radical optionally substituted by one or more halogen atoms, by one or more alkyl radicals having from 1 to 6 carbon atoms or by one or more alkoxy radicals having from 1 to 6 carbon atoms),
a radical of the formula $A_3$:

$-NHCONHR_4$ $(A_3)$

(in which $R_4$ represents an alkyl radical having from 1 to 6 carbon atoms or a phenyl radical optionally substituted by one or more halogen atoms, by one or more alkyl or alkoxy radicals having from 1 to 6 carbon atoms),
an o-sulphobenzoic imido radical of the formula $A_5$:

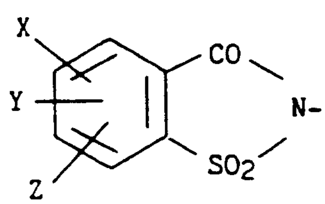

$(A_5)$

or a radical of the formula $A_5$:

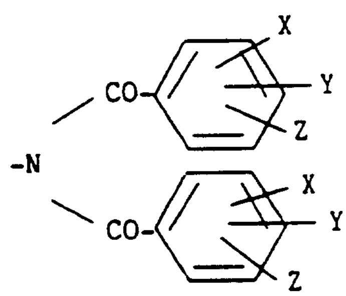

$(A_6)$

(in each of which X, Y and Z, which may be the same or different, each represent a hydrogen atom, a halogen atom, an alkyl radical having from 1 to 6 carbon atoms or an alkoxy radical having from 1 to 6 carbon atoms),
or a radical of the formula $A_7$:

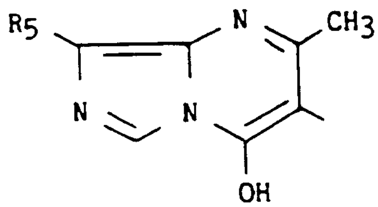

$(A_7)$

(in which $R_5$ represents a carbamoyl radical, a cyano radical, a carboxy radical, or an alkoxycarbonyl radical having from 1 to 6 carbon atoms),
or a radical of the formula $A_8$:

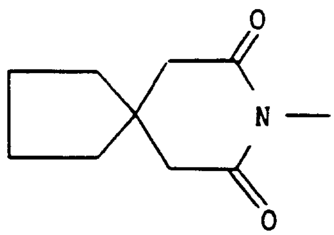

$(A_8)$,

their stereoisomers and their addition salts with a pharmaceutically acceptable mineral or organic acid.

**2.** 1-(7-methoxynaphth-1-yl)-4-[2-(4-fluorobenzoylamino)-ethyl]piperazine, a compound corresponding to formula I according to claim 1, and its addition salts with a pharmaceutically acceptable mineral or organic acid.

**3.** 1-(7-hydroxynaphth-1-yl)-4-[-2-(4-fluorobenzoylamino)-ethyl]piperazine, a compound corresponding to formula I according to claim 1, and its addition salts with a pharmaceutically acceptable mineral or organic acid.

**4.** 1-(7-methoxynaphth-1-yl)-4-[2-(thien-2-oylamino)-ethyl]piperazine, a compound corresponding to formula I according to claim 1, and its addition salts with a pharmaceutically acceptable mineral or organic acid.

**5.** 1-(7-methoxynaphth-1-yl-)-4-(2-butyrylaminoethyl)-piperazine, a compound corresponding to formula I according to claim 1, and its addition salts with a pharmaceutically acceptable mineral or organic acid.

**6.** Process for the preparation of compounds of the general formula I according to claim 1, characterised in that there is used as starting material:
**a/ either**
a compound of formula II:

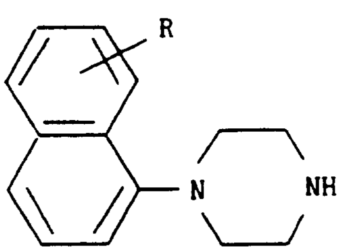

(II),

in which R is as defined for formula I according to claim 1,
which is condensed
either
with a nitrile of formula III:

$Hal(CH_2)_{n-1}CN$ (III),

in which Hal represents a halogen atom and n is as defined for formula I according to claim 1, in an organic solvent, at room temperature, in the presence of an alkali metal salt, to obtain the compounds of formula IV:

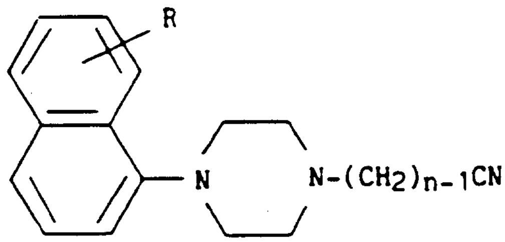

(IV),

in which R and n are as defined for formula I according to claim 1, which are converted with the aid of lithium aluminium hydride or another equivalent chemical reagent into a compound of formula V:

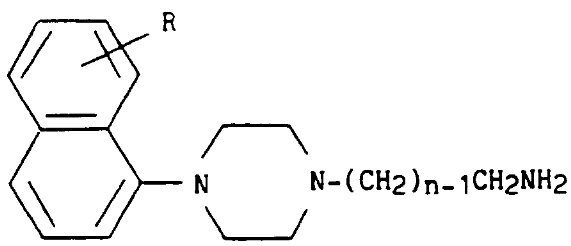

(V),

in which R and n are as defined for formula I according to claim 1,
which is reacted
**either**
with an equimolar amount of a compound of formula $VI_A$ or$VI_B$:

$ClCOR_2$ ($VI_A$)

$ClSO_2R_3$ ($VI_B$),

in which $R_2$ and $R_3$ are as defined for formula I according to claim 1, to obtain, respectively, the compounds of formula I in which $R_1$ represents a radical of the formula $A_1$ and the compounds of formula I in which $R_1$ represents a radical of the formula $A_2$,
which compounds of formula I, in which $R_2$ represents an -O-alkyl radical, are reacted, if desired, with a compound of formula VII:

$R_4NH_2$ (VII),

in which $R_4$ is as defined for formula I according to claim 1,
to form the compounds of formula I in which $R_1$ represents a radical of the formula $A_3$,
**or**
with a compound of formula VIII, in excess:

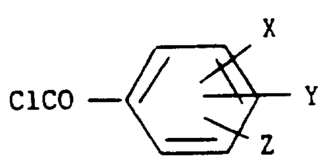

(VIII),

in which X, Y and Z are as defined for formula I according to claim 1,
to form a compound of formula I according to claim 1 in which $R_1$ represents a radical of the formula

$A_6$,
or
with a compound of formula $IX_B$:

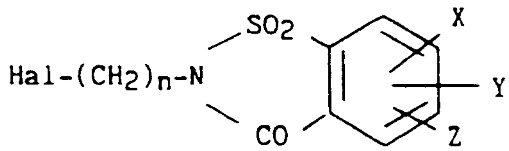

($IX_B$),

in which Hal is as defined for formula III and X, Y and Z are as defined for formula I according to claim 1,
to form the compounds of formula I according to claim 1 in which $R_1$ is a radical of the formula $A_6$,
or
with an alcohol of formula X:

$Hal(CH_2)_nOH$ (X),

in which n is as defined for formula I according to claim 1 and Hal is as defined for formula III,
to form the compounds of formula XI:

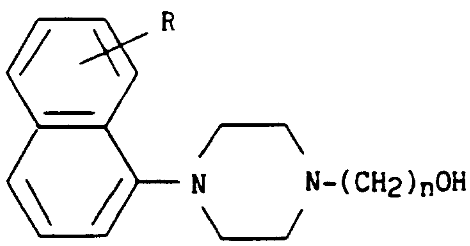

(XI),

in which R and n are as defined for formula I according to claim 1,
which are subjected to the action of thionyl chloride or another equivalent chemical reagent to form the compounds of formula XII:

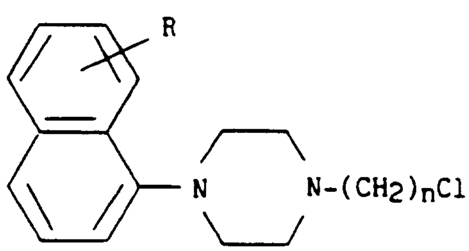

(XII),

in which R and n are as defined for formula I according to claim 1,
which are condensed
**either**
with ethyl acetoacetate to form a compound of formula XIII:

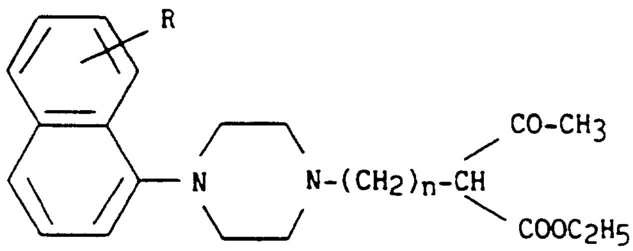

(XIII),

in which n and R are as defined for formula I according to claim 1, which is condensed with a 4-aminoimidazole compound of formula XIV:

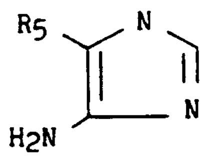

(XIV),

in which $R_5$ is as defined for formula I according to claim 1, to form the compounds of formula I according to claim 1 in which $R_1$ represents a radical of the formula $A_7$,

**or**

with a compound of formula XV:

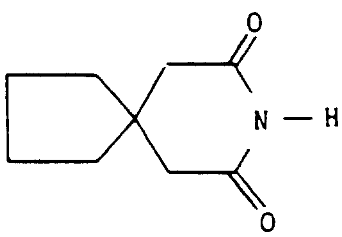

(XV)

to form the compounds of formula I in which $R_1$ represents a radical of the formula $A_8$,

**b/ or**

a compound of formula XVI:

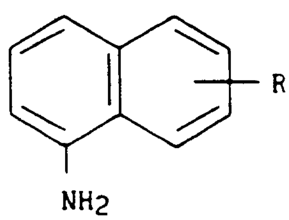

(XVI),

in which R is as defined in formula I, which is condensed with N-benzyliminodiacetic acid of formula XVII, which has previously been refluxed with carbonyldiimidazole in an anhydrous solvent such as tetrahydrofuran:

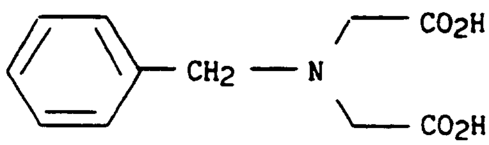

(XVII)

to yield a piperazine-2,6-dione of formula XVIII:

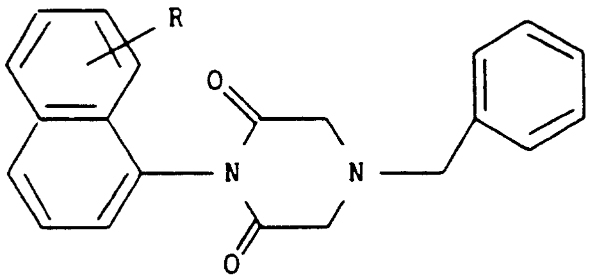

(XVIII),

in which R is as defined in formula I,
which is subjected to catalytic hydrogenation in the presence of palladium-on-carbon as catalyst, to yield a piperazine-2,6-dione of formula XIX:

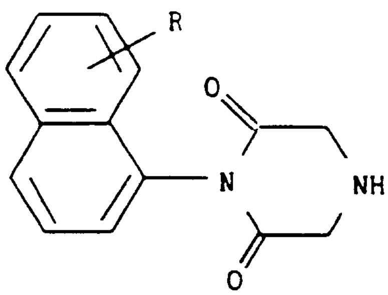

(XIX),

in which R is as defined in formula I,
which is condensed
either
with a nitrile of formula III:

$Hal-(CH_2)_{n-1}-CN$ (III),

in which Hal and n are as defined above,
to yield a piperazine-2,6-dione of formula XX:

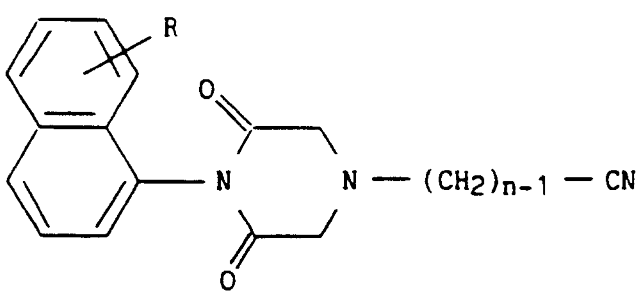

(XX),

in which R and n are as defined in formula I,
which is reduced in the presence of the borane-dimethyl sulphide complex to yield a piperazine of

formula V:

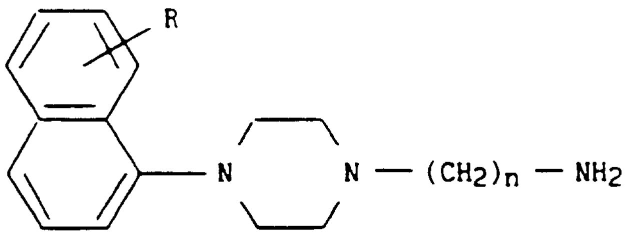

(V),

in which R and n are as defined in formula I,
which is reacted
**either**
with an equimolar amount of a compound of formula $VI_A$ or$VI_B$:

$ClCOR_2$ ($VI_A$)

$ClSO_2R_3$ ($VI_B$),

in which $R_2$ and $R_3$ are as defined for formula I, to obtain, respectively, the compounds of formula I in which $R_1$ represents a radical of the formula $A_1$ and the compounds of formula I in which $R_1$ represents a radical of the formula $A_2$,
which compounds of formula I, in which $R_2$ represents an -O-alkyl radical, are reacted, if desired, with a compound of formula VII:

$R_4NH_2$ (VII),

in which $R_4$ is as defined for formula I,
to form the compounds of formula I in which $R_1$ represents a radical of the formula $A_3$,
**or**
with a compound of formula VIII, in excess:

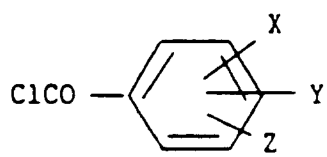

(VIII),

in which X, Y and Z are as defined for formula I,
to form a compound of formula I in which $R_1$ represents a radical of the formula $A_6$,
or
with an alcohol of formula X:

$Hal(CH_2)_nOH$ (X),

in which Hal and n are as defined above,
to yield a piperazine-2,6-dione of formula XXI:

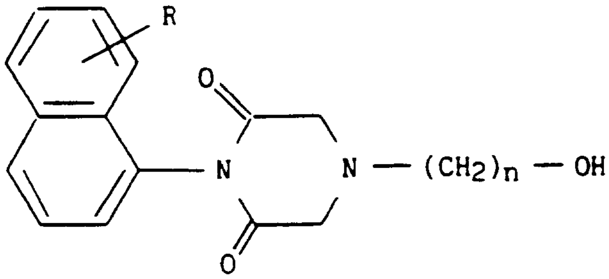

(XXI),

in which R and n are as defined in formula I, which is subjected to the action of thionyl chloride or another equivalent chemical reagent, to yield a piperasine-2,6-dione of formula XXII:

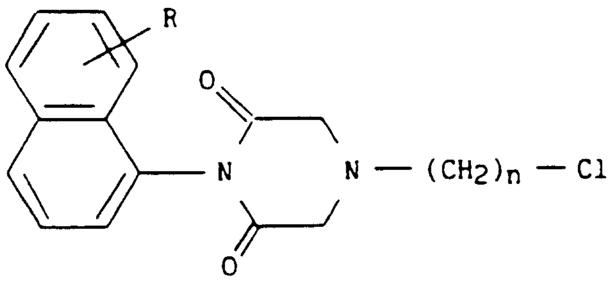

(XXII),

in which R and n are as defined in formula I, which is reduced in the presence of the borane-dimethyl sulphide complex to yield a piperazine of formula XII:

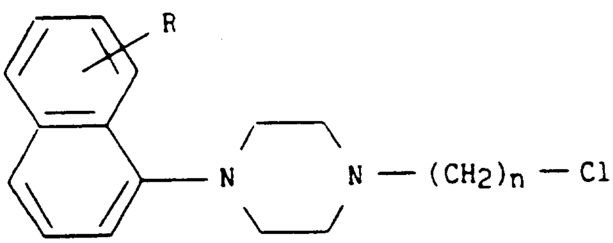

(XII),

in which R and n are as defined in formula I, which is condensed

**either**

with a compound of formula $XXIII_B$ or XV

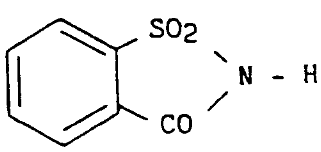

($XXIII_B$)

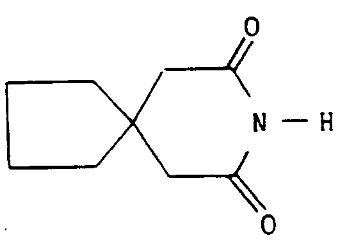

(XV)

to yield, respectively, the compounds of formula I in which $R_1$ is a radical $A_5$ or $A_5$,

**or**
with ethyl acetoacetate to form a compound of formula XIII:

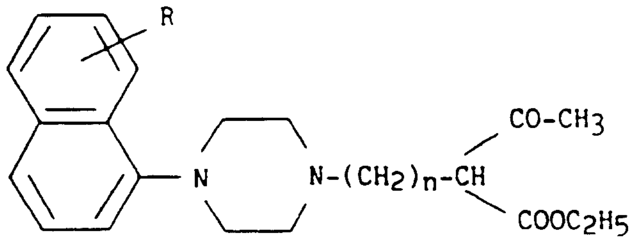

(XIII),

in which n and R are as defined for formula I,
which is condensed with a 4-aminoimidazole compound of formula XIV:

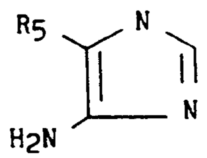

(XIV),

in which $R_5$ is as defined for formula I,
to form the compounds of formula I in which $R_1$ represents a radical of the formula $A_7$,
which compounds of formula I are then,
if desired, converted into salts with a pharmaceutically acceptable organic or mineral acid to form the corresponding addition salts.

**7.** Pharmaceutical composition containing as active ingredient a compound according to any one of claims 1 to 4, in association or in admixture with a pharmaceutically acceptable inert, non-toxic excipient or carrier.

**8.** Pharmaceutical composition according to claim 6 containing the active ingredient in a dose of from 0.05 to 10 mg.

**9.** Pharmaceutical composition according to claims 6 and 7 containing as active ingredient at least one compound according to claims 1 to 4, for use in the treatment of diseases requiring $5\text{-}HT_{1A}$ receptor agonists or antagonists.

**10.** Pharmaceutical composition according to claims 6 and 7 containing as active ingredient at least one compound according to claims 1 to 4, for use as an antihypertensive, an anxiolytic and an antidepressant.

**Claims for the following Contracting States : ES, GR**

**1.** Process for the preparation of compounds of formula I:

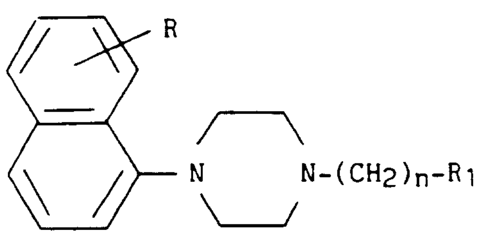

(I)

in which:

- n represents an integer from 1 to 4;
- R represents a hydrogen atom, a halogen atom, an alkyl radical having from 1 to 6 carbon atoms, a hydroxy radical, or an alkoxy radical having from 1 to 6 carbon atoms; and
- $R_1$ represents a radical of the formula $A_1$:

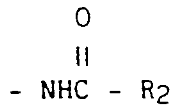

($A_1$)

(in which $R_2$ represents an alkyl radical having from 1 to 6 carbon atoms, a cycloalkyl radical having from 3 to 7 carbon atoms, a phenyl radical optionally substituted by one or more halogen atoms, by one or more alkyl radicals having from 1 to 6 carbon atoms or by one or more alkoxy radicals having from 1 to 6 carbon atoms, an -O-alkyl radical having from 1 to 6 carbon atoms, a 3-pyridyl radical, a 2-pyrrolyl radical, a quinolyl radical, a 1-isoquinolyl radical, a 2-thienyl radical or a 3-indolyl radical),
a radical of the formula $A_2$:

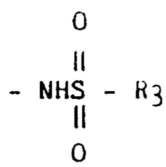

($A_2$)

(in which $R_3$ represents an alkyl radical having from 1 to 6 carbon atoms, a cycloalkyl radical having from 3 to 7 carbon atoms or a phenyl radical optionally substituted by one or more halogen atoms, by one or more alkyl radicals having from 1 to 6 carbon atoms or by one or more alkoxy radicals having from 1 to 6 carbon atoms),
a radical of the formula $A_3$:

$-NHCONHR_4$ ($A_3$)

(in which $R_4$ represents an alkyl radical having from 1 to 6 carbon atoms or a phenyl radical optionally substituted by one or more halogen atoms, by one or more alkyl or alkoxy radicals having from 1 to 6 carbon atoms),
an o-sulphobenzoic imido radical of the formula $A_5$:

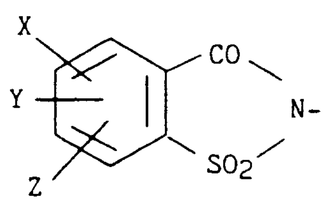

($A_5$)

or a radical of the formula $A_5$:

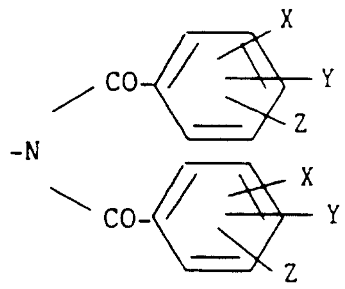

($A_6$)

(in each of which X, Y and Z, which may be the same or different, each represent a hydrogen atom, a halogen atom, an alkyl radical having from 1 to 6 carbon atoms or an alkoxy radical having from 1 to 6 carbon atoms),
or a radical of the formula $A_7$:

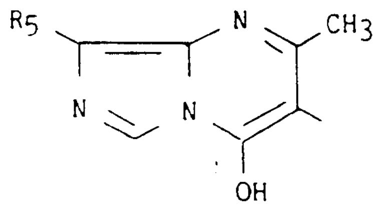

($A_7$)

(in which $R_5$ represents a carbamoyl radical, a cyano radical, a carboxy radical, or an alkoxycarbonyl radical having from 1 to 6 carbon atoms),
or a radical of the formula $A_8$:

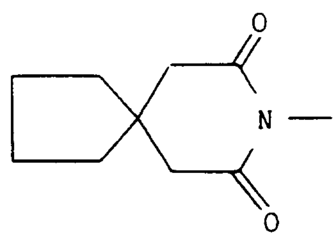

($A_8$),

their stereoisomers and their addition salts with a pharmaceutically acceptable mineral or organic acid, characterised in that there is used as starting material:

**a/ either**

a compound of formula II:

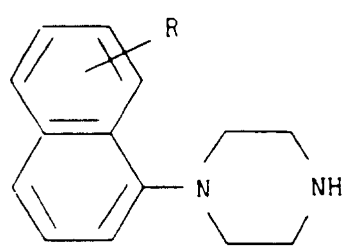

(II),

in which R is as defined for formula I,
which is condensed
either
with a nitrile of formula III:

$Hal(CH_2)_{n-1}CN$ (III),

in which Hal represents a halogen atom and n is as defined for formula I, in an organic solvent, at room temperature, in the presence of an alkali metal salt, to obtain the compounds of formula IV:

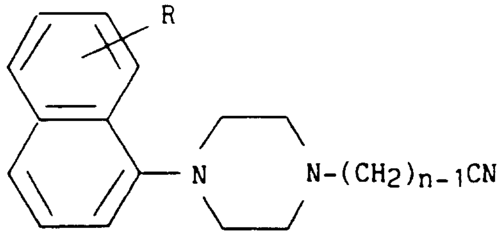

(IV),

in which R and n are as defined for formula I,

which are converted with the aid of lithium aluminium hydride or another equivalent chemical reagent into a compound of formula V:

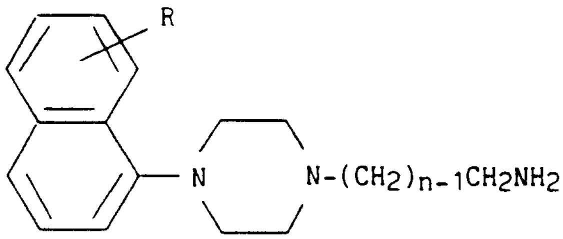

(V),

in which R and n are as defined for formula I according to claim 1,
which is reacted
**either**
with an equimolar amount of a compound of formula $VI_A$ or$VI_B$:

$ClCOR_2$ $(VI_A)$

$ClSO_2R_3$ $(VI_B)$,

in which $R_2$ and $R_3$ are as defined for formula L,

to obtain, respectively, the compounds of formula I in which $R_1$ represents a radical of the formula $A_1$ and the compounds of formula I in which $R_1$ represents a radical of the formula $A_2$,
which compounds of formula I, in which $R_2$ represents an -O-alkyl radical, are reacted, if desired, with a compound of formula VII:

$R_4NH_2$ (VII),

in which $R_4$ is as defined for formula I according to claim 1,
to form the compounds of formula I in which $R_1$ represents a radical of the formula $A_3$,
**or**
with a compound of formula VIII, in excess:

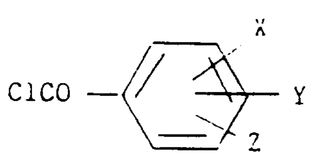

(VIII),

in which X, Y and Z are as defined for formula I according to claim 1,
to form a compound of formula I in which $R_1$ represents a radical of the formula $A_6$,
or

with a compound of formula $IX_B$:

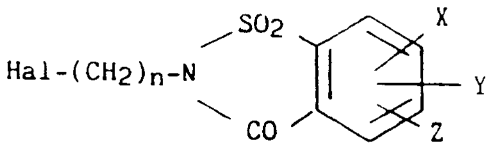

($IX_B$),

in which Hal is as defined for formula III and X, Y and Z are as defined for formula I, to form, respectively, the compounds of formula I according to claim 1 in which $R_1$ is a radical of the formula $A_5$,

or

with an alcohol of formula X:

$Hal(CH_2)_nOH$ (X),

in which n is as defined for formula I according to claim 1 and Hal is as defined for formula III, to form the compounds of formula XI:

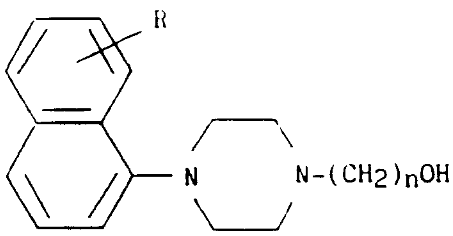

(XI),

in which R and n are as defined for formula I, which are subjected to the action of thionyl chloride or another equivalent chemical reagent to form the compounds of formula XII:

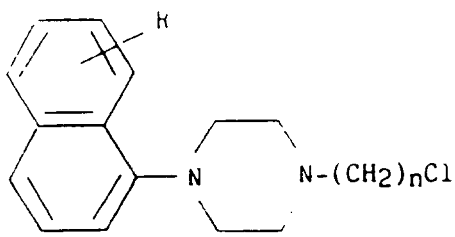

(XII),

in which R and n are as defined for formula I, which are condensed

**either**

with ethyl acetoacetate to form a compound of formula XIII:

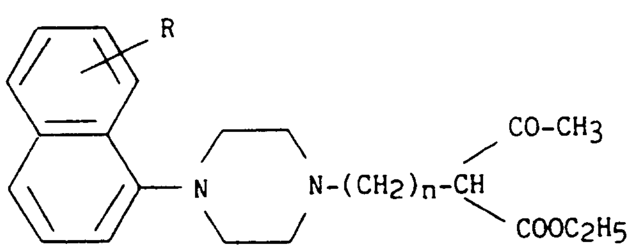

(XIII),

in which n and R are as defined for formula I according to claim 1,

which is condensed with a 4-aminoimidazole compound of formula XIV:

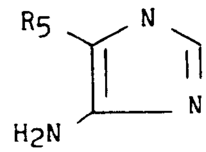

(XIV),

in which $R_5$ is as defined for formula I,
to form the compounds of formula I
in which $R_1$ represents a radical of the formula $A_7$,
**or**
with a compound of formula XV:

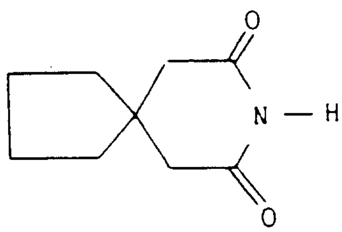

(XV)

to form the compounds of formula I in which $R_1$ represents a radical of the formula $A_8$,
**b/ or**
a compound of formula XVI:

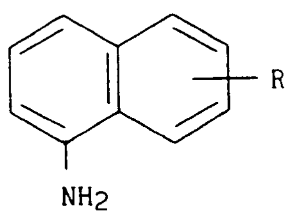

(XVI),

in which R is as defined in formula I,
which is condensed with N-benzyliminodiacetic acid of formula XVII, which has previously been refluxed with carbonyldiimidazole in an anhydrous solvent such as tetrahydrofuran:

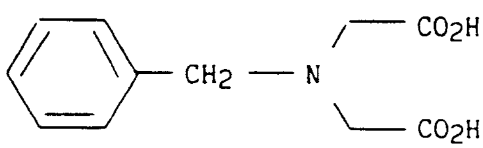

(XVII)

to yield a piperazine-2,6-dione of formula XVIII:

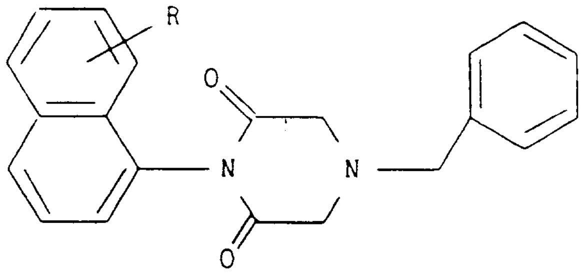

(XVIII),

in which R is as defined in formula I,
which is subjected to catalytic hydrogenation in the presence of palladium-on-carbon as catalyst, to yield a piperazine-2,6-dione of formula XIX: in which R is as defined in formula I,
which is condensed
either
with a nitrile of formula III:

$Hal-(CH_2)_{n-1}-CN$ (III),

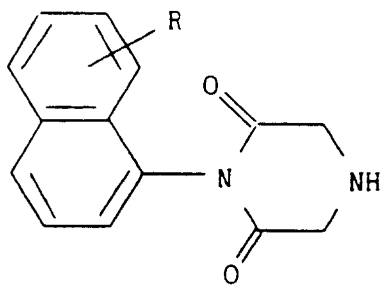

(XIX),

in which Hal and n are as defined above,
to yield a piperazine-2,6-dione of formula XX:

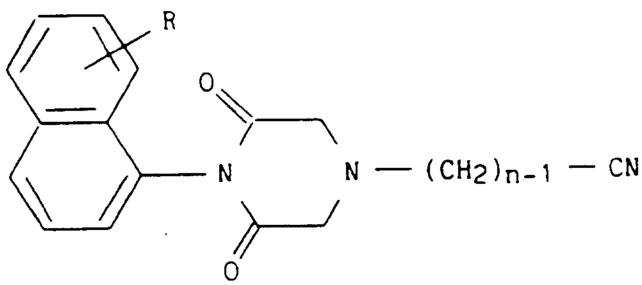

(XX),

in which R and n are as defined in formula I,
which is reduced in the presence of the borane-dimethyl sulphide complex to yield a piperazine of formula V:

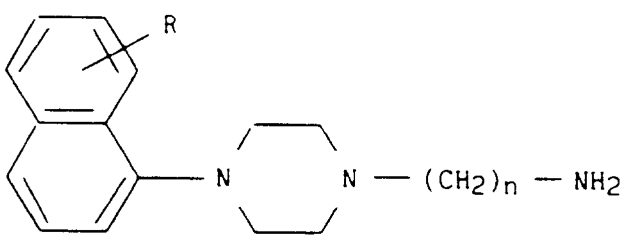

(V),

in which R and n are as defined in formula I,
which is reacted
**either**
with an equimolar amount of a compound of formula $VI_A$ or$VI_B$:

$ClCOR_2$ ($VI_A$)

$ClSO_2R_3$ ($VI_B$),

in which $R_2$ and $R_3$ are as defined for formula I, to obtain, respectively, the compounds of formula I in which $R_1$ represents a radical of the formula $A_1$ and the compounds of formula I in which $R_1$ represents a radical of the formula $A_2$,
which compounds of formula I, in which $R_2$ represents an -O-alkyl radical, are reacted, if desired, with a compound of formula VII:

$R_4NH_2$ (VII),

in which $R_4$ is as defined for formula I,
to form the compounds of formula I in which $R_1$ represents a radical of the formula $A_3$,
**or**
with a compound of formula VIII, in excess:

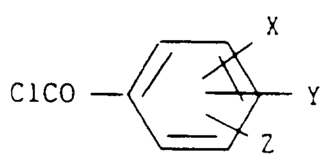
(VIII),

in which X, Y and Z are as defined for formula I,
to form a compound of formula I in which $R_1$ represents a radical of the formula $A_6$,
or
with an alcohol of formula X:

$Hal(CH_2)_nOH$ (X),

in which Hal and n are as defined above,
to yield a piperazine-2,6-dione of formula XXI:

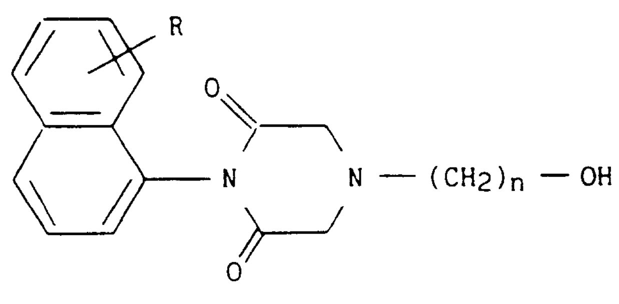
(XXI),

in which R and n are as defined in formula I,
which is subjected to the action of thionyl chloride or another equivalent chemical reagent,
to yield a piperazine-2,6-dione of formula XXII:

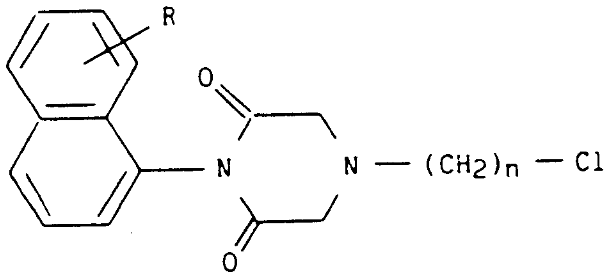

(XXII),

in which R and n are as defined in formula I,
which is reduced in the presence of the borane-dimethyl sulphide complex to yield a piperazine of formula XII: in which R and n are as defined in formula I,
which is condensed

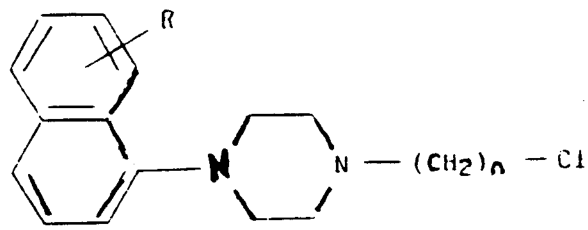

(XII),

**either**
with a compound of formula $XXIII_B$ or XV

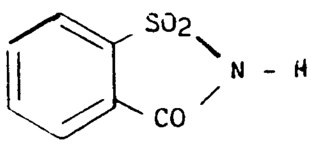

($XXIII_B$)

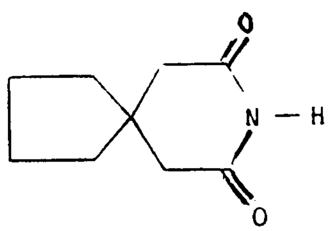

(XV) .

to yield, respectively, the compounds of formula I in which $R_1$ is a radical $A_5$ or $A_5$,
**or**
with ethyl acetoacetate to form a compound of formula XIII:

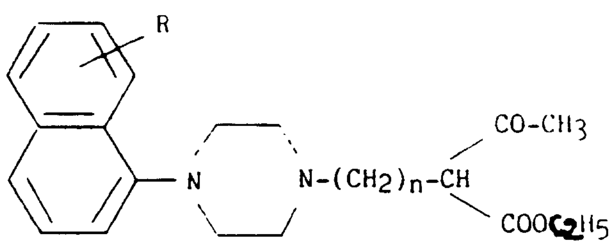

(XIII),

in which n and R are as defined for formula I,
which is condensed with a 4-aminoimidazole compound of formula XIV:

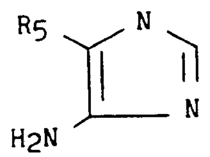

(XIV),

in which $R_5$ is as defined for formula I,
to form the compounds of formula I in which $R_1$ represents a radical of the formula $A_7$,
which compounds of formula I are then,
if desired, converted into salts with a pharmaceutically acceptable organic or mineral acid to form the corresponding addition salts.

2. Process according to claim 1 for the preparation of 1-(7-methoxynaphth-1-yl)-4-[2-(4-fluorobenzoylamino)-ethyl]piperazine and its addition salts with a pharmaceutically acceptable mineral or organic acid.

3. Process according to claim 1 for the preparation of 1-(7-hydroxynaphth-1-yl)-4-[2-(4-fluorobenzoylamino)-ethyl]piperazine and its addition salts with a pharmaceutically acceptable mineral or organic acid.

4. Process according to claim 1 for the preparation of 1-(7-methoxynaphth-1-yl)-4-[2-(thien-2-oylamino)-ethyl]-piperazine and its addition salts with a pharmaceutically acceptable mineral or organic acid.

5. Process according to claim 1 for the preparation of 1-(7-methoxynaphth-1-yl)-4-(2-butyrylaminoethyl)-piperazine and its addition salts with a pharmaceutically acceptable mineral or organic acid.

**Patentansprüche**

**Patentansprüche für folgende Vertragsstaaten : AT, BE, CH, DE, DK, FR, GB, IT, LI, LU, NL, SE**

1. Verbindungen der Formel I:

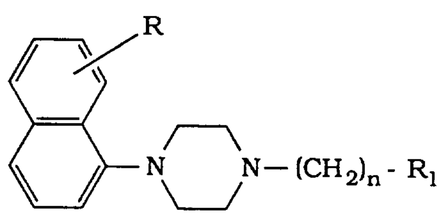

(I)

in der:
- n eine ganze Zahl mit einem Wert von 1 bis 4,
- R ein Wasserstoffatom, ein Halogenatom, eine Alkylgruppe mit 1 bis 6 Kohlenstoffatomen, eine Hydroxylgruppe oder eine Alkoxygruppe mit 1 bis 6 Kohlenstoffatomen,
- $R_1$ eine Gruppe der Formel $A_1$:

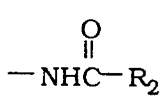

($A_1$)

(in der
- $R_2$ eine Alkylgruppe mit 1 bis 6 Kohlenstoffatomen, eine Cycloalkylgruppe mit 3 bis 7 Kohlenstoffatomen, eine Phenylgruppe - die gegebenenfalls durch eines oder mehrere Halogenatome oder eine oder mehrere Alkylgruppen mit 1 bis 6 Kohlenstoffatomen oder eine oder mehrere Alkoxygruppen mit 1 bis 6 Kohlenstoffatomen substituiert ist - eine -O-Alkylgruppe mit 1 bis 6 Kohlenstoffatomen, eine 3-Pyridylgruppe, eine 2-Pyrrolylgruppe, eine Chinolylgruppe, eine 1-Isochinolylgruppe, eine 2-Thienylgruppe oder eine 3-Indolylgruppe darstellt), eine Gruppe der Formel $A_2$:

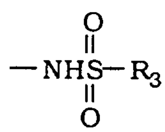

($A_2$)

(in der $R_3$ eine Alkylgruppe mit 1 bis 6 Kohlenstoffatomen, eine Cycloalkylgruppe mit 3 bis 7 Kohlenstoffatomen oder eine Phenylgruppe - die gegebenenfalls durch eines oder mehrere Halogenatome, eine oder mehrere Alkylgruppen mit 1 bis 6 Kohlenstoffatomen oder eine oder mehrere Alkoxygruppen mit 1 bis 6 Kohlenstoffatomen substituiert ist - darstellt),

eine Gruppe der Formel $A_3$:

$- NHCONHR_4$ ($A_3$)

(in der $R_4$ eine Alkylgruppe mit 1 bis 6 Kohlenstoffatomen oder eine Phenylgruppe, die gegebenenfalls durch eines oder mehrere Halogenatome oder durch eine oder mehrere Alkyl- oder Alkoxygruppen mit 1 bis 6 Kohlenstoffatomen substituiert ist, darstellt),

eine O-Sulfobenzoeimidogruppe der Formel $A_5$:

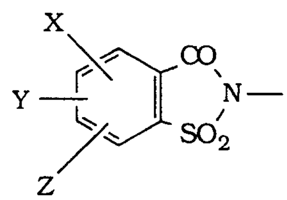

($A_5$)

oder eine Gruppe der Formel $A_5$:

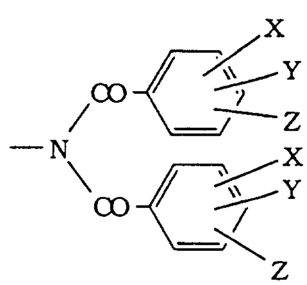

($A_6$)

(in denen X, Y und Z, die gleichartig oder verschieden sein können, jeweils ein Wasserstoffatom, ein Halogenatom, eine Alkylgruppe mit 1 bis 6 Kohlenstoffatomen oder eine Alkoxygruppe mit 1 bis 6 Kohlenstoffatomen darstellen),

oder eine Gruppe der Formel $A_7$:

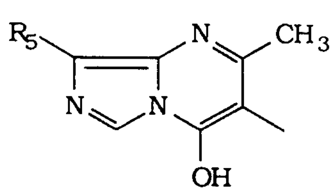

($A_7$)

(in der $R_5$ eine Carbamoylgruppe, eine Cyanogruppe, eine Carboxygruppe oder eine Alkoxycarbonylgruppe mit 1 bis 6 Kohlenstoffatomen darstellt),

oder eine Gruppe der Formel $A_8$:

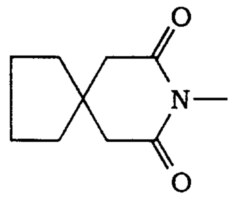

($A_8$)

bedeuten,

deren Stereoisomere und deren Additionssalze mit einer anorganischen oder organischen, pharmazeutisch annehmbaren Säure.

2. Verbindung der Formel I nach Anspruch 1, nämlich 1-(7-Methoxynaphth-1-yl)-4-[2-(4-fluorbenzoylamino)-ethyl]-piperazin und dessen Additionssalze mit einer anorganischen oder organischen, pharmazeutisch annehmbaren Säure.

3. Verbindung der Formel I nach Anspruch 1, nämlich 1-(7-Hydroxynaphth-1-yl)-4-[2-(4-fluorbenzoylamino)-ethyl]-piperazin und dessen Additionssalze mit einer anorganischen oder organischen, pharmazeutisch annehmbaren Säure.

4. Verbindung der Formel I nach Anspruch 1, nämlich 1-(7-Methoxynaphth-1-yl)-4-[2-(thien-2-oyl-amino)-ethyl]-piperazin und dessen Additionssalze mit einer anorganischen oder organischen, pharmazeutisch annehmbaren Säure.

5. Verbindung der Formel I nach Anspruch 1, nämlich 1-(7-Methoxynaphth-1-yl)-4-(2-butyrylaminoethyl)-piperazin und dessen Additionssalze mit einer anorganischen oder organischen, pharmazeutisch annehmbaren Säure.

6. Verfahren zur Herstellung der Verbindungen der allgemeinen Formel I nach Anspruch 1, **dadurch gekennzeichnet,** daß man als Ausgangsmaterial:

**a/ entweder**

eine Verbindung der Formel II:

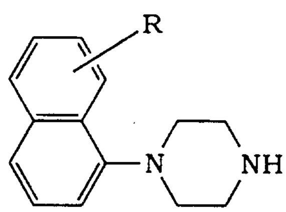

(II)

in der R die bezüglich der Formel I in Anspruch 1 angegebenen Bedeutungen besitzt, verwendet und

**entweder**

mit einem Nitril der Formel III:

$Hal(CH_2)_{n-1}CN$ (III)

in der Hal ein Halogenatom darstellt und n die bezüglich der Formel I in Anspruch 1 angegebenen Bedeutungen besitzt, in einem organischen Lösungsmittel bei Raumtemperatur in Gegenwart eines Alkalimetallsalzes kondensiert zur Bildung der Verbindungen der Formel IV:

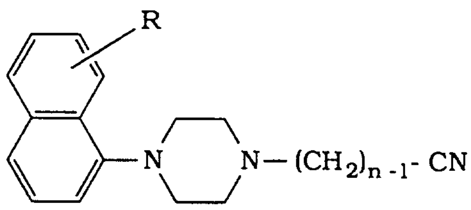

(IV)

in der R und n die bezüglich der Formel I in Anspruch 1 angegebenen Bedeutungen besitzen, welche man mit Lithiumaluminiumhydrid oder einem anderen chemisch äquivalenten Reagens in eine Verbindung der Formel V:

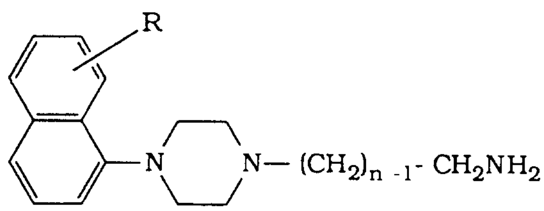

(V)

in der R und n die bezüglich der Formel I in Anspruch 1 angegebenen Bedeutungen besitzen, umwandelt.

die man

entweder

mit einer äquimolaren Menge einer Verbindung der Formel $VI_A$ oder $VI_B$:

$ClCOR_2$ ($VI_A$)

$ClSO_2R_3$ ($VI_B$)

worin $R_2$ und $R_3$ die bezüglich der Formel I in Anspruch 1 angegebenen Bedeutungen besitzen, umsetzt zur Bildung der Verbindungen der Formel I, worin $R_1$ eine Gruppe der Formel $A_1$ darstellt, bzw. der Verbindungen der Formel I, worin $R_1$ eine Gruppe der Formel $A_2$ darstellt,

welche Verbindungen der Formel I, worin $R_2$ eine -O-Alkylgruppe darstellt, man gewünschtenfalls mit einer Verbindung der Formel VII:

$R_4NH_2$ (VII)

worin $R_4$ die bezüglich der Formel I in Anspruch 1 angegebenen Bedeutungen besitzt, umsetzt

zur Bildung der Verbindungen der Formel I, worin $R_1$ eine Gruppe der Formel $A_3$ darstellt,

oder

mit einer im Überschuß eingesetzten Verbindung der Formel VIII:

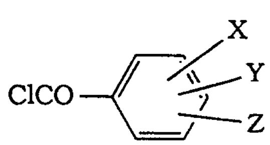

(VIII)

in der X, Y und Z die bezüglich der Formel I in Anspruch 1 angegebenen Bedeutungen besitzen,

umsetzt zur Bildung einer Verbindung der Formel I nach Anspruch 1, worin $R_1$ eine Gruppe der Formel $A_6$ darstellt,

**oder**

mit einer Verbindung der Formel $IX_B$:

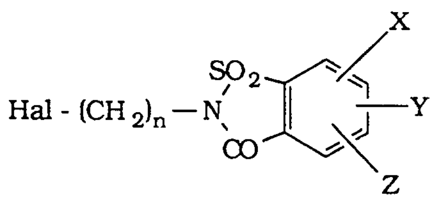

($IX_B$)

in der Hal die bezüglich der Formel III angegebenen Bedeutungen besitzt und X, Y und Z die bezüglich der Formel I in Anspruch 1 angegebenen Bedeutungen besitzen,

kondensiert zur Bildung der Verbindungen der Formel I nach Anspruch 1, worin $R_1$ eine Gruppe der Formel $A_5$ darstellt,

**oder**

mit einem Alkohol der Formel X:

$Hal(CH_2)_nOH$ (X)

in der n die bezüglich der Formel I in Anspruch 1 angegebenen Bedeutungen besitzt und Hal die bezüglich der Formel III angegebenen Bedeutungen aufweist, kondensiert

zur Bildung der Verbindungen der Formel XI:

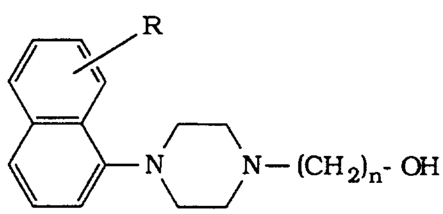

(XI)

in der R und n die bezüglich der Formel I in Anspruch 1 angegebenen Bedeutungen besitzen,

welche man der Einwirkung von Thionylchlorid oder eines anderen chemisch äquivalenten Reagens unterwirft zur Bildung der Verbindungen der Formel XII:

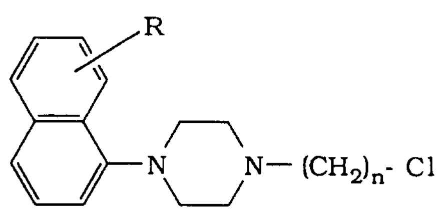

(XII)

in der R und n die bezüglich der Formel I in Anspruch 1 angegebenen Bedeutungen besitzen,

welche man

entweder

mit Acetessigsäureethylester kondensiert zur Bildung einer Verbindung der Formel XIII:

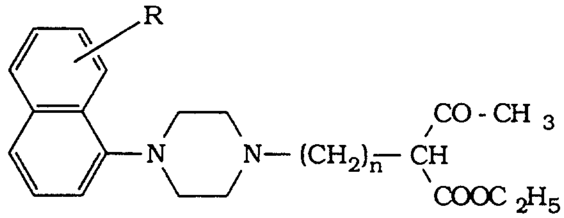

(XIII)

in der n und R die bezüglich der Formel I in Anspruch 1 angegebenen Bedeutungen besitzen, welche man mit einem 4-Amino-imidazolderivat der Formel XIV:

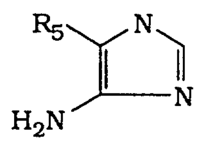

(XIV)

in der $R_5$ die bezüglich der Formel I in Anspruch 1 angegebenen Bedeutungen besitzt, kondensiert zur Bildung der Verbindungen der Formel I nach Anspruch 1, worin $R_1$ eine Gruppe der Formel $A_7$ darstellt,

oder

mit einer Verbindung der Formel XV:

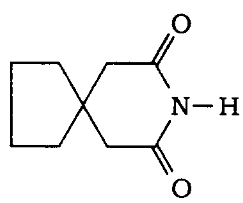

(XV)

kondensiert zur Bildung der Verbindungen der Formel I, worin $R_1$ eine Gruppe der Formel $A_8$ darstellt,

**b/ oder**

eine Verbindung der Formel XVI:

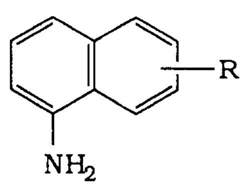

(XVI)

in der R die bezüglich der Formel I angegebenen Bedeutungen besitzt, verwendet, die man mit N-Benzyliminodiessigsäure der Formel XVII, die man zuvor mit Carbonyldiimidazol In einem wasserfreien Lösungsmittel, wie Tetrahydrofuran, zum Sieden am Rückfluß erhitzt hat, kondensiert

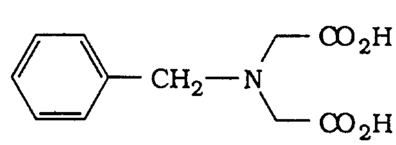

(XVII)

zur Bildung eines Piperazin-2,6-dions der Formel XVIII:

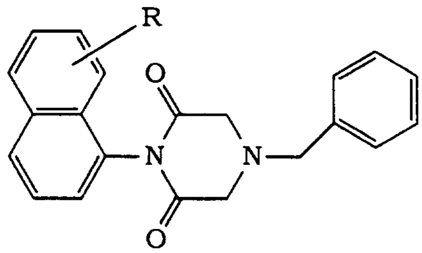

(XVIII)

in der R die bezüglich der Formel I angegebenen Bedeutungen besitzt, welches man einer katalytischen Hydrierung in Gegenwart von Palladium-auf-Kohlenstoff als Katalysator hydriert,

zur Bildung eines Piperazin-2,6-dions der Formel XIX:

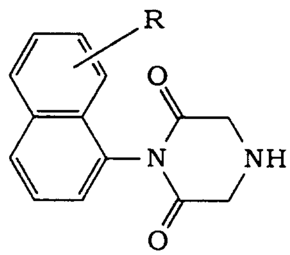

(XIX)

in der R die bezüglich der Formel I angegebenen Bedeutungen besitzt, welches man

**entweder**

mit einem Nitril der Formel III:

$Hal-(CH_2)_{n-1}-CN$ (III)

in der Hal und n die oben angegebenen Bedeutungen besitzen, kondensiert zur Bildung eines Piperazin-2,6-dions der Formel XX:

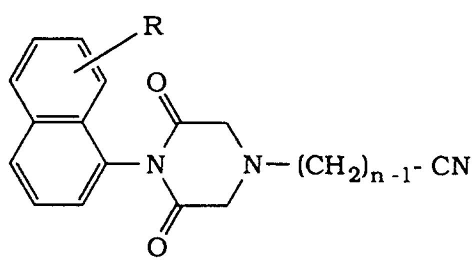

(XX)

in der R und n die bezüglich der Formel I angegebenen Bedeutungen besitzen,
welches man in Gegenwart eines Boran-Dimethylsulfid-Komplexes reduziert
zur Bildung eines Piperazins der Formel V:

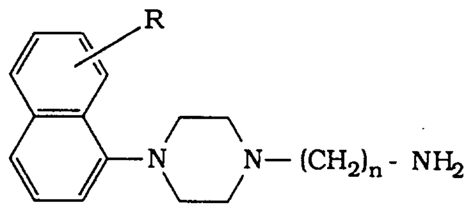

(V)

in der R und n die bezüglich der Formel I angegebenen Bedeutungen besitzen, welches man

entweder

mit einer äquimolaren Menge einer Verbindung der Formel $VI_A$ oder $VI_B$:

Cl CO $R_2$ ($VI_A$)

Cl $SO_2$ $R_3$ ($VI_B$)

worin $R_2$ und $R_3$ die bezüglich der Formel 1 angegebenen Bedeutungen besitzen, umsetzt, so daß man die Verbindungen der Formel I, worin $R_1$ eine Gruppe der Formel $A_1$ darstellt, bzw. der Verbindungen der Formel I, worin $R_1$ eine Gruppe der Formel $A_2$ darstellt, erhält,

welche Verbindungen der Formel I, worin $R_2$ eine -O-Alkylgruppe darstellt, man gewünschtenfalls mit einer Verbindung der Formel VII:

$R_4NH_2$ (VII)

in der $R_4$ die bezüglich der Formel I angegebenen Bedeutungen besitzt, umsetzt zur Bildung der Verbindungen der Formel I, in der $R_1$ eine Gruppe der Formel $A_3$ darstellt,

oder

mit einer im Überschuß eingesetzten Verbindung der Formel VIII;

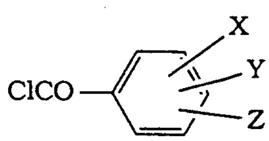

(VIII)

in der X, Y und Z die bezüglich der Formel I angegebenen Bedeutungen besitzen, umsetzt zur Bildung einer Verbindung der Formel I, in der $R_1$ eine Gruppe der Formel $A_6$ darstellt,

**oder**

mit einem Alkohol der Formel X:

Hal ($CH_2$)n OH (X)

in der Hal und n die oben angegebenen Bedeutungen besitzen, kondensiert zur Bildung eines Piperazin-2,6-dions der Formel XXI:

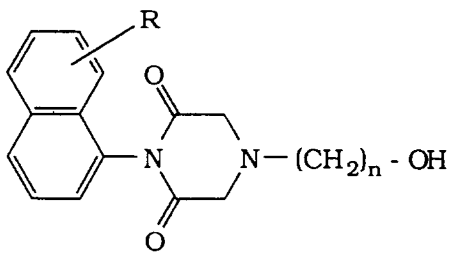

(XXI)

in der R und n die bezüglich der Formel I angegebenen Bedeutungen besitzen,

weiches man der Einwirkung von Thionylchlorid oder eines anderen chemisch äquivalenten Reagens unterwirft,

zur Bildung eines Piperazin-2,6-dions der Formel XXII:

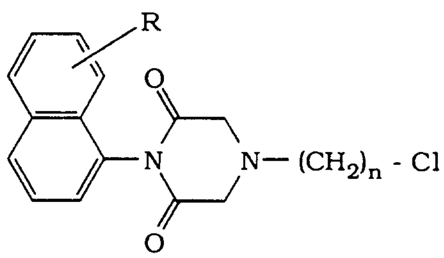

(XXII)

in der R und n die bezüglich der Formel I angegebenen Bedeutungen besitzen,

welches man in Gegenwart des Boran-Dimethylsulfid-Komplexes reduziert

zur Bildung eines Piperazins der Formel XII:

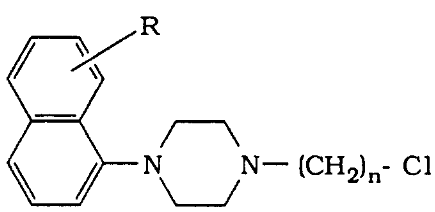

(XII)

in der R und n die bezüglich der Formel I angegebenen Bedeutungen besitzen,

welches man

entweder

mit einer Verbindung der Formel $XXIII_B$ oder XV:

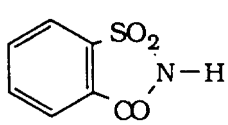

($XXIII_B$)

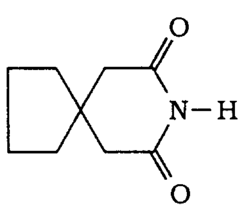

(XV)

kondensiert, so daß man die Verbindungen der Formel I, in der $R_1$ eine Gruppe $A_5$ darstellt bzw. eine Gruppe $A_5$ darstellt, erhält,

oder

mit Acetessigsäureethylester kondensiert zur Bildung einer Verbindung der Formel XIII:

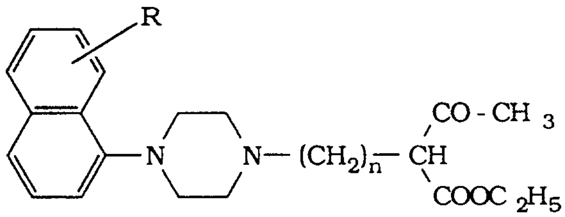

(XIII)

in der n und R die bezüglich der Formel I angegebenen Bedeutungen besitzen,
welche man mit einem 4-Amino-imidazolderivat der Formel XIV:

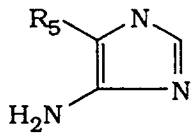

(XIV)

in der $R_5$ die bezüglich der Formel I angegebenen Bedeutungen besitzt, kondensiert
zur Bildung der Verbindungen der Formel I, in der $R_1$ eine Gruppe der Formel $A_7$ darstellt,
**welche Verbindungen der Formel I anschließend**
gewünschtenfalls mit einer organischen oder anorganischen, pharmazeutisch annehmbaren Säure in ihre entsprechenden Additionssalze überführt werden.

7. Pharmazeutische Zubereitung enthaltend als Wirkstoff eine Verbindung nach einem der Ansprüche 1 bis 5 in Kombination oder in Mischung mit einem inerten, nichttoxischen, pharmazeutisch annehmbaren Trägermaterial oder Bindemittel.

8. Pharmazeutische Zubereitung nach Anspruch 7, enthaltend den Wirkstoff in einer Dosis von 0,05 bis 10 mg.

9. Pharmazeutische Zubereitung nach den Ansprüchen 7 und 8, enthaltend als Wirkstoff mindestens eine Verbindung nach den Ansprüchen 1 bis 5 zur Behandlung von Erkrankungen, welche die Anwendung von Agonisten oder Antagonisten der $5\text{-HT}_{1A}$-Rezeptoren erforderlich machen.

10. Pharmazeutische Zubereitung nach den Ansprüchen 7 und 8, enthaltend als Wirkstoff mindestens eine Verbindung nach den Ansprüchen 1 bis 5 zur Verwendung als Antihypertensivum, Anxiolytikum und Antidepressivum.

**Patentansprüche für folgende Vertragsstaaten : ES, GR**

1. Verfahren zur Herstellung der Verbindungen der Formel I:

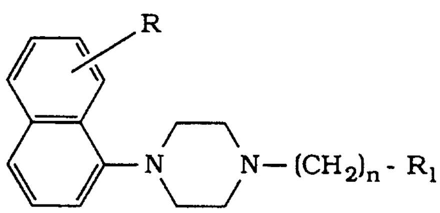

(I)

in der:

- n eine ganze Zahl mit einem Wert von 1 bis 4.

- R ein Wasserstoffatom, ein Halogenatom, eine Alkylgruppe mit 1 bis 6 Kohlenstoffatomen, eine Hydroxylgruppe oder eine Alkoxygruppe mit 1 bis 6 Kohlenstoffatomen,
- $R_1$ eine Gruppe der Formel $A_1$:

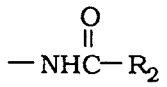

$(A_1)$

(in der
- $R_2$ eine Alkylgruppe mit 1 bis 6 Kohlenstoffatomen, eine Cycloalkylgruppe mit 3 bis 7 Kohlenstoffatomen, eine Phenylgruppe - die gegebenenfalls durch eines oder mehrere Halogenatome oder eine oder mehrere Alkylgruppen mit 1 bis 6 Kohlenstoffatomen oder eine oder mehrere Alkoxygruppen mit 1 bis 6 Kohlenstoffatomen substituiert ist - eine -O-Alkylgruppe mit 1 bis 6 Kohlenstoffatomen, eine 3-Pyridylgruppe, eine 2-Pyrrolylgruppe, eine Chinolylgruppe, eine 1-Isochinolylgruppe, eine 2-Thienylgruppe oder eine 3-Indolylgruppe darstellt),

eine Gruppe der Formel $A_2$:

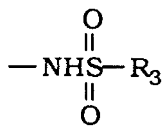

$(A_2)$

(in der $R_3$ eine Alkylgruppe mit 1 bis 6 Kohlenstoffatomen, eine Cycloalkylgruppe mit 3 bis 7 Kohlenstoffatomen oder eine Phenylgruppe - die gegebenenfalls durch eines oder mehrere Halogenatome, eine oder mehrere Alkylgruppen mit 1 bis 6 Kohlenstoffatomen oder eine oder mehrere Alkoxygruppen mit 1 bis 6 Kohlenstoffatomen substituiert ist - darstellt),

eine Gruppe der Formel $A_3$:

- $NHCONHR_4$ $(A_3)$

(in der $R_4$ eine Alkylgruppe mit 1 bis 6 Kohlenstoffatomen oder eine Phenylgruppe, die gegebenenfalls durch eines oder mehrere Halogenatome oder durch eine oder mehrere Alkyl- oder Alkoxygruppen mit 1 bis 6 Kohlenstoffatomen substituiert ist, darstellt),

eine O-Sulfobenzoeimidogruppe der Formel $A_5$:

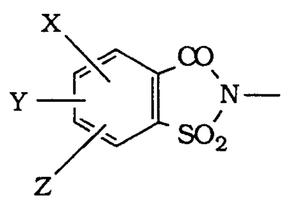

$(A_5)$

oder eine Gruppe der Formel $A_5$:

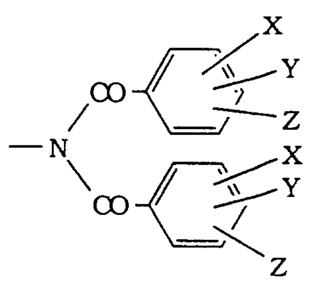

$(A_6)$

(in denen X, Y und Z, die gleichartig oder verschieden sein können, jeweils ein Wasserstoffatom, ein Halogenatom, eine Alkylgruppe mit 1 bis 6 Kohlenstoffatomen oder eine Alkoxygruppe mit 1 bis 6 Kohlenstoffatomen darstellen),

oder eine Gruppe der Formel $A_7$:

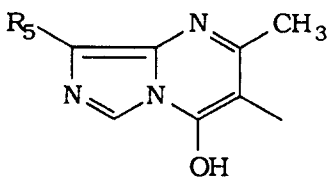

($A_7$)

(in der $R_5$ eine Carbamoylgruppe, eine Cyanogruppe, eine Carboxygruppe oder eine Alkoxycarbonylgruppe mit 1 bis 6 Kohlenstoffatomen darstellt),

oder eine Gruppe der Formel $A_8$:

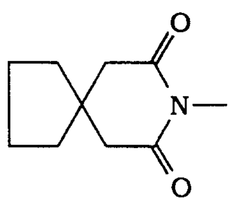

($A_8$)

bedeuten,

und von deren Stereoisomeren und deren Additionssalzen mit einer anorganischen oder organischen, pharmazeutisch annehmbaren Säure, **dadurch gekennzeichnet,** daß man als Ausgangsmaterial:

**a/ entweder**

eine Verbindung der Formel II:

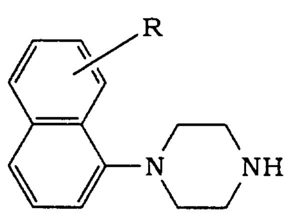

(II)

in der R die bezüglich der Formel I angegebenen Bedeutungen besitzt, verwendet und

**entweder**

mit einem Nitril der Formel III:

$Hal(CH_2)_{n-1}CN$ (III)

in der Hal ein Halogenatom darstellt und n die bezuglich der Formel I angegebenen Bedeutungen besitzt, in einem organischen Lösungsmittel bei Raumtemperatur in Gegenwart eines Alkalimetallsalzes kondensiert zur Bildung der Verbindungen der Formel IV:

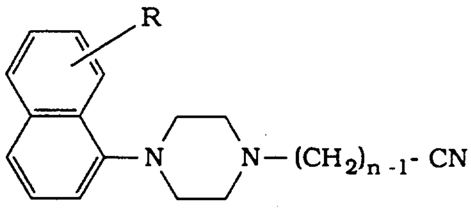

(IV)

in der R und n die bezüglich der Formel I angegebenen Bedeutungen besitzen, welche man mit Lithiumaluminiumhydrid oder einem anderen chemisch äquivalenten Reagens in eine Verbindung der Formel V:

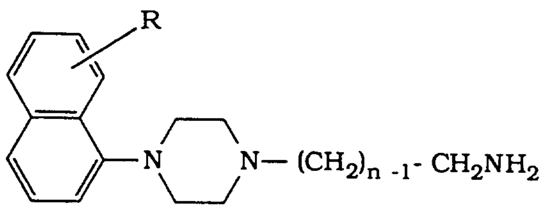

(V)

in der R und n die bezüglich der Formel I angegebenen Bedeutungen besitzen, umwandelt,
die man
entweder
mit einer äquimolaren Menge einer Verbindung der Formel $VI_A$ oder $VI_B$:

$ClCOR_2$ ($VI_A$)

$ClSO_2R_3$ ($VI_B$)

worin $R_2$ und $R_3$ die bezüglich der Formel 1 angegebenen Bedeutungen besitzen, umsetzt zur Bildung der Verbindungen der Formel I, worin $R_1$ eine Gruppe der Formel $A_1$ darstellt, bzw. der Verbindungen der Formel I, worin $R_1$ eine Gruppe der Formel $A_2$ darstellt,
welche Verbindungen der Formel I, worin $R_2$ eine -O-Alkylgruppe darstellt, man gewünschtenfalls mit einer Verbindung der Formel VII:

$R_4NH_2$ (VII)

worin $R_4$ die bezüglich der Formel angegebenen Bedeutungen besitzt, umsetzt
zur Bildung der Verbindungen der Formel I, worin $R_1$ eine Gruppe der Formel $A_3$ darstellt,
oder
mit einer im Überschuß eingesetzten Verbindung der Formel VIII:

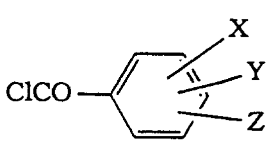

(VIII)

in der X, Y und Z die bezüglich der Formel I angegebenen Bedeutungen besitzen,
umsetzt zur Bildung einer Verbindung der Formel I , worin $R_1$ eine Gruppe der Formel $A_6$ darstellt,

**oder**

mit einer Verbindung der Formel $IX_B$:

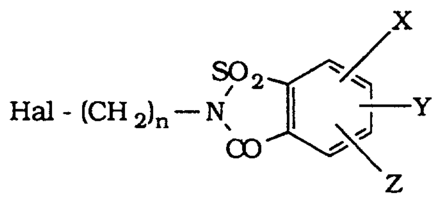

$(IX_B)$

in der Hal die bezüglich der Formel III angegebenen Bedeutungen besitzt und X, Y und Z die bezüglich der Formel I angegebenen Bedeutungen besitzen,

kondensiert zur Bildung der Verbindungen der Formel I, worin $R_1$ eine Gruppe der Formel $A_5$ darstellt,

**oder**

mit einem Alkohol der Formel X:

$Hal(CH_2)_nOH$ (X)

in der n die bezüglich der Formel I angegebenen Bedeutungen besitzt und Hal die bezüglich der Formel III angegebenen Bedeutungen aufweist, kondensiert

zur Bildung der Verbindungen der Formel XI:

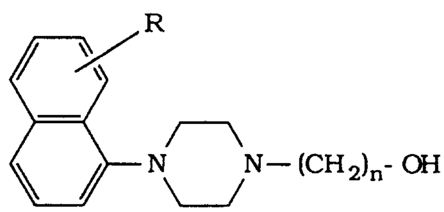

(XI)

in der R und n die bezüglich der Formel I angegebenen Bedeutungen besitzen, welche man der Einwirkung von Thionylchlorid oder eines anderen che

misch äquivalenten Reagens unterwirft zur Bildung der Verbindungen der Formel XII:

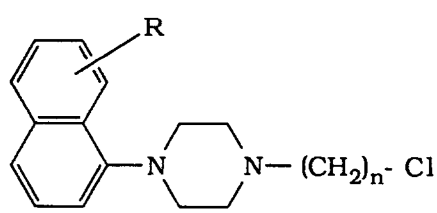

(XII)

in der R und n die bezüglich der Formel I angegebenen Bedeutungen besitzen,

welche man

entweder

mit Acetessigsäureethylester kondensiert zur Bildung einer Verbindung der Formel XIII:

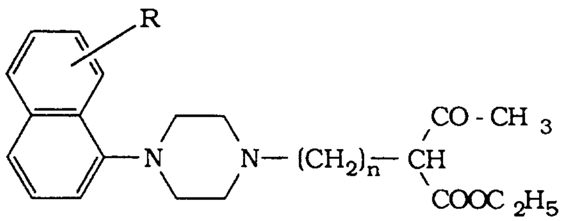

(XIII)

in der n und R die bezüglich der Formel I angegebenen Bedeutungen besitzen, welche man mit einem 4-Amino-imidazolderivat der Formel XIV:

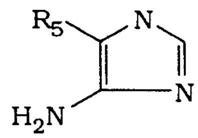

(XIV)

in der $R_5$ die bezüglich der Formel I angegebenen Bedeutungen besitzt, kondensiert zur Bildung der Verbindungen der Formel I, worin $R_1$ eine Gruppe der Formel $A_7$ darstellt,

oder

mit einer Verbindung der Formel XV:

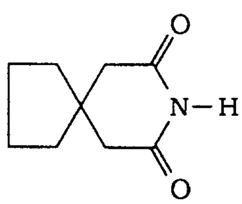

(XV)

kondensiert zur Bildung der Verbindungen der Formel I, worin $R_1$ eine Gruppe der Formel $A_8$ darstellt,

**b/ oder**

eine Verbindung der Formel XVI:

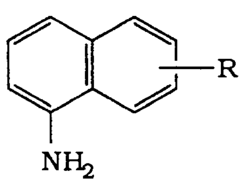

(XVI)

in der R die bezüglich der Formel I angegebenen Bedeutungen besitzt, verwendet, die man mit N-Benzyliminodiessigsäure der Formel XVII, die man zuvor mit Carbonyldiimidazol in einem wasserfreien Lösungsmittel, wie Tetrahydrofuran, zum Sieden am Rückfluß erhitzt hat, kondensiert

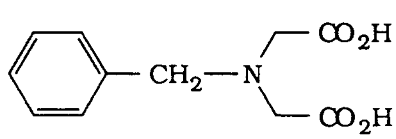

(XVII)

zur Bildung eines Piperazin-2,6-dions der Formel XVIII:

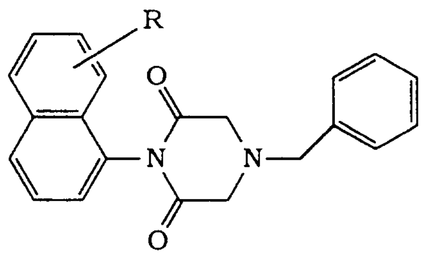

(XVIII)

in der R die bezüglich der Formel I angegebenen Bedeutungen besitzt, welches man einer katalytischen Hydrierung in Gegenwart von Palladium-auf-Kohlenstoff als Katalysator hydriert,

zur Bildung eines Piperazin-2,6-dions der Formel XIX:

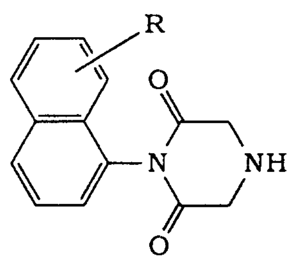

(XIX)

in der R die bezüglich der Formel I angegebenen Bedeutungen besitzt, welches man

**entweder**

mit einem Nitril der Formel III:

$Hal\text{-}(CH_2)_{n-1}\text{ - }CN$ (III)

in der Hal und n die oben angegebenen Bedeutungen besitzen, kondensiert zur Bildung eines Piperazin-2,6-dions der Formel XX:

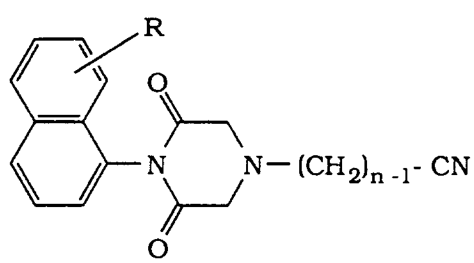

(XX)

in der R und n die bezüglich der Formel I angegebenen Bedeutungen besitzen,
welches man in Gegenwart eines Boran-Dimethylsulfid-Komplexes reduziert
zur Bildung eines Piperazins der Formel V:

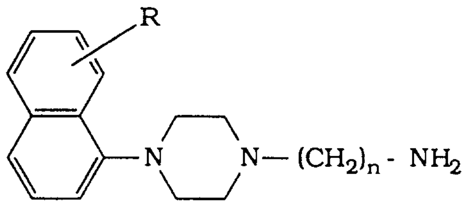

(V)

in der R und n die bezüglich der Formel I angegebenen Bedeutungen besitzen,
welches man

entweder

mit einer äquimolaren Menge einer Verbindung der Formel $VI_A$ oder $VI_B$:

Cl CO $R_2$ ($VI_A$)

Cl $SO_2$ $R_3$ ($VI_B$)

worin $R_2$ und $R_3$ die bezüglich der Formel 1 angegebenen Bedeutungen besitzen, umsetzt, so daß man die Verbindungen der Formel I, worin $R_1$ eine Gruppe der Formel $A_1$ darstellt, bzw. der Verbindungen der Formel I, worin $R_1$ eine Gruppe der Formel $A_2$ darstellt, erhält,

welche Verbindungen der Formel I, worin $R_2$ eine -O-Alkylgruppe darstellt, man gewünschtenfalls mit einer Verbindung der Formel VII:

$R_4NH_2$ (VII)

in der $R_4$ die bezuglich der Formel I angegebenen Bedeutungen besitzt, umsetzt
zur Bildung der Verbindungen der Formel I, in der $R_1$ eine Gruppe der Formel $A_3$ darstellt,

oder

mit einer im Überschuß eingesetzten Verbindung der Formel VIII:

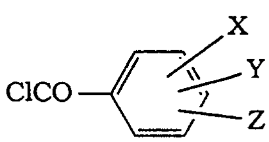

(VIII)

in der X, Y und Z die bezüglich der Formel I angegebenen Bedeutungen besitzen, umsetzt
zur Bildung einer Verbindung der Formel I, in der $R_1$ eine Gruppe der Formel $A_6$ darstellt,

**oder**

mit einem Alkohol der Formel X:

Hal $(CH_2)_n$ OH (X)

in der Hal und n die oben angegebenen Bedeutungen besitzt, kondensiert
zur Bildung eines Piperazin-2,6-dions der Formel XXI:

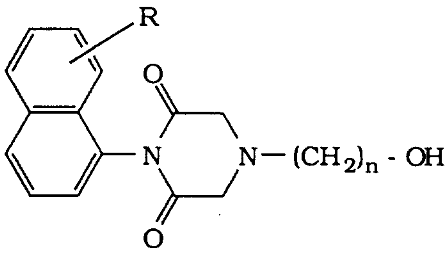

(XXI)

in der R und n die bezüglich der Formel I angegebenen Bedeutungen besitzen,

welches man der Einwirkung von Thionylchlorid oder eines anderen chemisch äquivalenten Reagens unterwirft,

zur Bildung eines Piperazin-2,6-dions der Formel XXII:

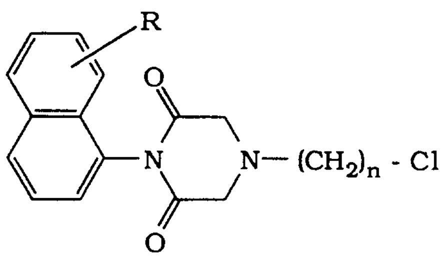

(XXII)

in der R und n die bezüglich der Formel I angegebenen Bedeutungen besitzen,

welches man in Gegenwart des Boran-Dimethylsulfid-Komplexes reduziert

zur Bildung eines Piperazins der Formel XII:

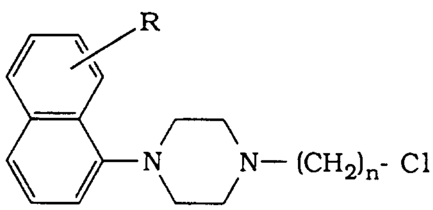

(XII)

in der R und n die bezüglich der Formel I angegebenen Bedeutungen besitzen,

welches man

entweder

mit einer Verbindung der Formel $XXIII_B$ oder XV:

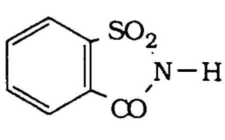

($XXIII_B$)

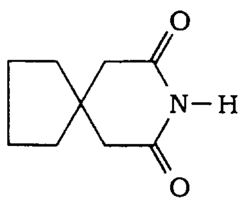

(XV)

kondensiert, so daß man die Verbindungen der Formel I, in der $R_1$ eine Gruppe $A_5$ darstellt bzw. eine Gruppe $A_5$ darstellt, erhält,

oder

mit Acetessigsäureethylester kondensiert zur Bildung einer Verbindung der Formel XIII:

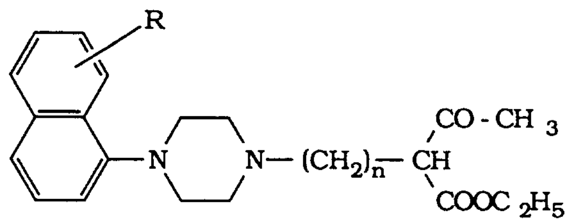

(XIII)

in der n und R die bezüglich der Formel I angegebenen Bedeutungen besitzen, welche man mit einem 4-Amino-imidazolderivat der Formel XIV:

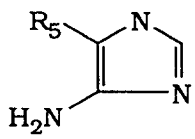

(XIV)

in der $R_5$ die bezüglich der Formel I angegebenen Bedeutungen besitzt, kondensiert zur Bildung der Verbindungen der Formel I, in der $R_1$ eine Gruppe der Formel $A_7$ darstellt,

**welche Verbindungen der Formel I anschließend**

gewünschtenfalls mit einer organischen oder anorganischen, pharmazeutisch annehmbaren Säure in ihre entsprechenden Additionssalze überführt werden.

**2.** Verfahren nach Anspruch 1 zur Herstellung von 1-(7-Methoxynaphth-1-yl)-4-[2-(4-fluorbenzoylamino)-ethyl]-piperazin und dessen Additionssalzen mit einer anorganischen oder organischen, pharmazeutisch annehmbaren Säure.

**3.** Verfahren nach Anspruch 1 zur Herstellung von 1-(7-Hydroxynaphth-1-yl)-4-[2-(4-fluorbenzoylamino)-ethyl]-piperazin und dessen Additionssalzen mit einer anorganischen oder organischen, pharmazeutisch annehmbaren Säure.

**4.** Verfahren nach Anspruch 1 zur Herstellung von 1-(7-Methoxynaphth-1-yl)-4-[2-(thien-2-oyl-amino)-ethyl]-piperazin und dessen Additionssalzen mit einer anorganischen oder organischen, pharmazeutisch annehmbaren Säure.

**5.** Verfahren nach Anspruch 1 zur Herstellung von 1-(7-Methoxynaphth-1-yl)-4-(2-butyrylaminoethyl)-piperazin und dessen Additionssalzen mit einer anorganischen oder organischen, pharmazeutisch annehmbaren Säure.